(19) Europäisches Patentamt
European Patent Office
Office européen des brevets

(11) Publication number: 0 209 751 B1

(12) # EUROPEAN PATENT SPECIFICATION

(45) Date of publication of patent specification: **16.09.92**

(51) Int. Cl.5: **C07D 501/24**, A61K 31/545

(21) Application number: **86108659.3**

(22) Date of filing: **25.06.86**

The file contains technical information submitted after the application was filed and not included in this specification

(54) **Cephalosporin derivatives and bactericides containing the same.**

(30) Priority: **26.06.85 JP 140989/85**
**26.10.85 JP 240276/85**

(43) Date of publication of application:
**28.01.87 Bulletin 87/05**

(45) Publication of the grant of the patent:
**16.09.92 Bulletin 92/38**

(84) Designated Contracting States:
**DE FR GB IT**

(56) References cited:

**CHEMICAL ABSTRACT, vol. 101, no. 25, 17th December 1984, page 743, abstract no. 230239t, Columbus, Ohio, US**

(73) Proprietor: **MEIJI SEIKA KAISHA LTD.**
**4-16 Kyobashi 2-chome**
**Chuo-ku Tokyo 104(JP)**

(72) Inventor: **Tsuruoka, Takashi**
**1-14-833, Kawaracho Saiwai-ku**
**Kawasaki-shi Kanagawa-ken(JP)**
Inventor: **Iwamatsu, Katsuyoshi**
**1-11-2-403, Serigaya Konan-ku**
**Yokohama-shi Kanagawa-ken(JP)**
Inventor: **Katano, Kiyoaki**
**2-67-1-705, Asahicho Tsurumi-ku**
**Yokohama-shi Kanagawa-ken(JP)**
Inventor: **Ogino, Hiroko Room 402**
**Musashikosugi View Heights 223-4, Ichinotsubo**
**Nakahara-ku Kawasaki-shi**
**Kanagawa-ken(JP)**
Inventor: **Okamoto, Ryoichi**
**1-18-16, Matsunami**
**Chiba-shi Chiba-ken(JP)**
Inventor: **Yoshida, Takashi**
**2-9-22, Himonya Meguro-ku**
**Tokyo(JP)**
Inventor: **Sezaki, Masaji**
**1-12-16, Sangenjaya Setagaya-ku**
**Tokyo(JP)**
Inventor: **Kai, Fumio**
**6-22-4, Shonandai**
**Fujisawa-shi Kanagawa-ken(JP)**
Inventor: **Inoue, Shigeharu**
**16-2, Tsutsujigaoka Midori-ku**
**Yokohama-shi Kanagawa-ken(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

Inventor: **Kondo, Shinichi**
**Room 801 Ichigao Annex 1157-1, Ichigao, Midori-ku**
**Yokohama-shi Kanagawa-ken(JP)**

(74) Representative: **Müller-Boré & Partner Patentanwälte**
**Isartorplatz 6 Postfach 26 02 47**
**W-8000 München 2(DE)**

## Description

### FIELD OF THE INVENTION

The present invetnion relates to new cephalosporin compounds. These compounds have a broad bactericidal spectrum against various bacteria including Pseudomonas aeruginosa and an excellent remedial effect for pathogenic diseases of human and animals and therefore are useful as medicines for human and animals.

### BACKGROUND OF THE INVENTION

Cephalosporin antibiotics are widely used for the remedy of diseases caused by pathogenic bacteria, and these are said to be effective for the remedy of diseases caused by such bacteria as tolerant to any other antibiotics such as penicillin-type antibiotics. Regarding the remedial effect of these antibiotics in clinical use, however, the effect can hardly be said sufficient even in the case of so-called cephem-type antibiotics of the third generation which have recently been developed as antibiotics against gram-negative bacteria including opportunistic pathogens.

For instance, cephalosporin derivatives in which the 2-amino group in the 7-aminothiazolylglycyl group is amidated with dihydroxybenzoic acid to form an amido-substituted compound are noted to have a strong bactericidal activity against Pseudomonas aeruginosa (refer to Japanese Patent Application OPI No. 139387/84; the term "OPI" as used herein means an "unexamined published application"), but these are defective as being easily O-methylated and deactivated in a living body.

Japanese Patent Application OPI No. 118792/84 discloses one example of cephalosporin antibiotics, in which the 2-amino group in the 7-aminothiazolylglycyl group is amidated into 5-hydroxy-4-pyridone-2-carboxamide derivative. However, this essentially discloses carbacephem-type antibiotics, and any concrete exmaples of 1,5-dihydroxy-4-pyridone-2-carboxamide substituents of the present invention are not described at all in said patent publication.

The synthesis of the 1,5-dihydroxy-4-pyridone-2-carboxylic acid, which is one constitutional element in the 7-positioned substituent of the derivatives of the present invention, is not so easy. This is because N-aminopyridone compounds can hardly be obtained by the reaction of a kojic acid and a hydrazine (refer to "Agr. Biol. Chem", Vol. 31, pp. 979-989, written by I.Ichimoto, et al. in 1967); and thus, when a pyrone ring is reacted with a hydroxyl-amine, not only the hydroxylamine-substitution on the intended oxygen atom but also the hydroxylamine-substitution on the carbonyl group is generally observed (refer to "Heterocyclic Compounds, Pyridine and its Derivatives", Vol. 14, Part 2, published by Interscience Publishers Ltd.).

Under the circumstances, the present inventors have earnesly studied various relevant techniques and have found that the said compound may be obtained in a relatively high yield by carrying out the reaction in the presence of pyridine or the like compound.

The compounds of the present invention are characterized by the existence of the 1,5-dihydroxy-4-pyridone-2-carboxamido group in the 7-positioned substituent therein; and the present compounds have an unexpectedly intensified and higher bactericidal activity against various bacteria, especially against Pseudomonas aeruginosa, than the known 5-hydroxy-4-pyridone-2-carboxamido-substitutedderivatives, and further, the present compounds have higher solubility in water, which is an important matter for injections. The present invention provides the new cephalosporin derivatives and concretely illustrates hereinafter the preparation and the use of these derivatives.

### SUMMARY OF THE INVENTION

The object of the present invention is to provide new cephalosporin antibiotic derivatives and pharmaceutically acceptable salts thereof, which are free from the defects of other conventional cephalosporin antibiotics.

Accordingly, the present invention provides new cephalosporin compounds as defined in claim 1 and pharmaceutically acceptable salts thereof as well as bactericides containing the same as an active ingredient as defined in claim 17.

### DETAILED DESCRIPTION OF THE INVENTION

The new cephalosporin compounds provided by the present invention are represented by the following general formula (I). Moreover, the invention provides pharmaceutically acceptable salts thereof as well as

bactericides containing the same as an active ingredient.

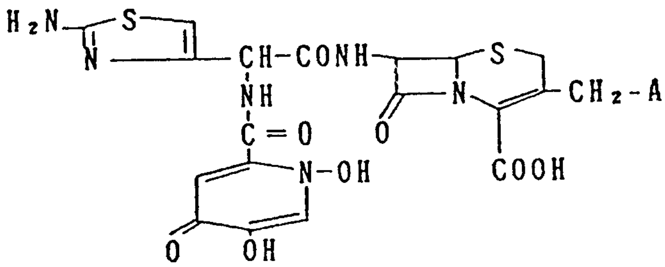

(I)

wherein A represents an alkanoyloxy group having 2-5 carbon atoms; a carbamoyloxy group; an azido group; or an unsubstituted or substituted pyridylthio group of a formula (I-1):

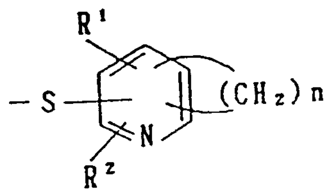

(I-1)

(where n is 0 or an integer of 3-5; $R^1$ and $R^2$ may be same or different and each represent a hydrogen atom, a halogen atom, a carboxyl group or an optionally halogen-substituted loweralkyl group having 1-5 carbon atoms); or an unsubstituted or substituted pyridiniumthio group of a formula (I-2):

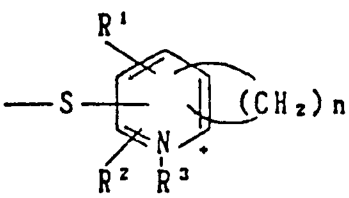

(I-2)

[where n, $R^1$ and $R^2$ have the same meanings as above; $R^3$ represents a linear or branched alkyl group having 1-5 carbon atoms, a halogen-substituted alkyl group, a cyclopropyl group, a cyclopropylmethyl group, an alkenyl group, an oxygen atom or a group of $-(CH_2)m-B$;
(m is an integer of 0-3; and B represents a hydroxyl group, an alkoxy group, an amino group, an alkyl-substituted amino group, a carboxyl group, a carbamoyl group, a sulfonic acid group, a sulfonic acid amide group, a hydroxamic acid group, a cyano group, a thiol group, an alkylthio group, a methanesulfonylaminocarbonyl group or an acetamidosulfonyl gorup)]; or an unsubstituted or substituted pyridinium group of a formula (I-3):

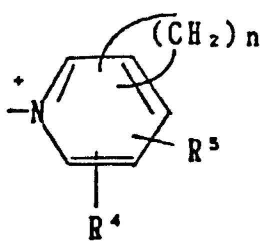

(I-3)

(where n has the same meaning as above; $R^4$ and $R^5$ may be same or different and each represent a hydrogen atom, a linear or branched alkyl group having 1-5 carbon atoms, a carboxyl group, a carbamoyl group, a sulfonic acid group, a sulfonic acid amide group, a linear or branched alkylthio group having 1-5 carbon atoms, a halogen-substituted alkylthio group, a cycloalkanothio group, a cycloalkanomethylthio group, a carboxyalkylthio group, a carbamoylalkylthio group, an alkoxyalkylthio group or an alkyl-substituted

aminoalkylthio group); or a 5- or 6-membered heterocyclicthio or bicycloheterocyclicthio group of a formula (I-4):

-S-Het (I-4)

(where Het represents an optionally substituted thiazole, isothiazole, 1,2,3-thiadiazole, 1,3,4-thiadiazole, 1,3,4-triazole, 1,2,3,4-tetrazole, pyrimidine, 1,2,4-triazine, benzothiazole, benzimidazole, benzoxazole, 1,3,4-triazaindolidine or 2,3-dihydro-1H-indolidinium group).

The α-carbon in the aminothiazolylglycyl substituent in the formula (I) may be in the form of D-isomer or L-isomer, and the present invention includes the both cases of D-isomer and L-isomer as well as DL-isomer.

In addition, the 1,5-dihydroxy-4-pyridone-2-carboxamido part in the 7-positioned substituent of the formula (I) may form the following tautomers, and the present invention includes both the two cases. The nomenclature and the structure of the compounds of the formula (I) are designated herein, based upon the corresponding pyridone-type compounds.

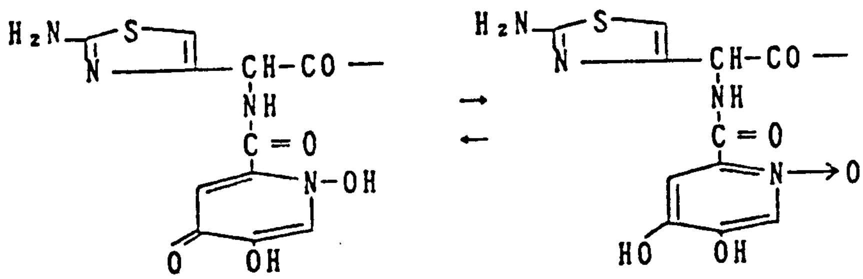

In the present invention, pharmaceutically acceptable salts of the compounds of the above formula (I) include medically acceptable salts, especially conventional non-toxic salts, for exmaple, inorganic salts with an inorganic base such as alkali metal salts (e.g. sodium or potassium salt), alkaline earth metal salts (e.g. calcium or magnesium salt) and ammonium salts, and organic salts with an organic base such as organic amine salts (e.g. triethylamine salt, pyridine salt, ethanolamine salt, triethanolamine salt or dicyclohexylamine salt) and basic amino acid salts (e.g. lysine or arginine salt).

Examples of the 3-positioned substituent of the compounds of the formula (I) of the present invention are given below, which, however, are not whatsoever limitative.

(Pyridin-4-yl)thiomethyl, (pyridin-3-yl)thiomethyl, (pyridin-2-yl)thiomethyl, (3-methoxypyridin-4-yl) thiomethyl, (2,3-dimethylpyridin-4-yl)thiomethyl, (2-carboxypyridin-4-yl)thiomethyl, (2-carbamoylpyridin-4-yl)thiomethyl, (2,3- cyclopentenopyridin-4-yl)thiomethyl, (pyridin-N-oxide-4-yl)thiomethyl, (2,3-cyclopentenopyridin-N-oxide-4-yl)thiomethyl, (5,6-cyclopentenopyridin-2-yl)thiomethyl, (2,3-cyclohexenopyridin-4-yl)thiomethyl, (5,6-cyclohexenopyridin-2-yl)thiomethyl, (1-methylpyridinium-4-yl)thiomethyl, (1-methylpyridinium-3-yl)-thiomethyl, (1-methylpyridinium-2-yl)thiomethyl, (1-ethylpyridinium-4-yl)thiomethyl, (1-allylpyridinium-4-yl) thiomethyl, [1-(2,2,2-trifluoroethyl) pyridinium-4-yl] thiomethyl, (1-carboxymethylpyridinium-4-yl)thiomethyl, (1-carbamoylmethylpyridinium-4-yl)thiomethyl, (1-hydroxyethylpyridinium-4-yl)thiomethyl, (1-dimethylaminoethylpyridinium-4-yl) thiomethyl, (1-cyclopropylpyridinium-4-yl)thiomethyl, (1-cyclopropylmethylpyridnium-4-yl)thiomethyl, (1-methylthiomethylpyridinium-4-yl)thiomethyl, (1-cyanomethylpyridinium-4-yl)thiomethyl, [1-(2-fluoroethyl)pyridinium-4-yl]thiomethyl, (1-hydroxyaminocarbonylmethylpyridinium-4-yl)thiomethyl, [1-(2-sulfoethyl)pyridinium-4-yl]thiomethyl, (1-sulfomethylpyridinium-4-yl)thiomethyl, [1-(2-sulfamoylethyl)pyridinium-4-yl]thiomethyl, (1-sulfamoylmethylpyridinium-4-yl)thiomethyl, (1-N,N-dimethylsulfamoylmethylpyridinium-4-yl)thiomethyl. [2-methyl-1-(2-hydroxyethyl)pyridinium-4-yl]thiomethyl, (2,6-dimethyl-1-carboxymethylpyridinium-4-yl)-thiomethyl, (3,5-dimethyl-1-carboxymethylpyridinium-4-yl)thiomethyl, (2-carboxy-1-methylpyridinium-4-yl)-thiomethyl, (2,3-dihydro-1H-indolidinium-5-yl)thiomethyl, (1-ethylpyridinium-3-yl)thiomethyl, (1-cyclopropylpyridinium-3-yl)thiomethyl, [1-(2-hydroxyethyl)pyridinium-3-yl]thiomethyl, (1-carboxymethylpyridinium-3-yl)thiomethy], (1-carbamoylmethylpyridinium-3-yl)thiomethyl, [1-(2-fluoroethyl)-pyridinium-3-yl] thiomethyl, [1-(2,2,2-trifluoroethyl)pyridinium-3-yl] thiomethyl, (1-sulfomethylpyridinium-3-yl)thiomethyl, (1-sulfamoylmethylpyridinium-3-yl)thiomethyl, [1-(2-sulfoethyl)pyridinium-3-yl]thiomethyl, (1-cyclopropylpyridinium-2-yl)thiomethyl, [1-(2-hydroxyethyl)pyridinium-2-yl]thiomethyl, (1-carboxymethylpyridinium-2-yl)thiomethyl, (1-carbamoylmethylpyridinium-2-yl)thiomethyl, (1-sulfomethylpyridinium-2-yl)thiomethyl, (2,3-cyclopenteno-1-methylpyridinium-4-yl)thiomethyl, (2,3-cyclopenteno-1-ethylpyridinium-4-yl)thiomethyl, (2,3-cyclopenteno-1-allylpyridinium-4-yl)thiomethyl, [2,3-cyclopenteno-1-(2,2,2-trifluoroethyl)pyridinium-4-yl]thiomethyl,(2,3-cyclopenteno-1-carboxymethylpyridinium-4-yl) thiomethyl, (2,3-cyclopenteno-1-carbamoylmethylpyridinium-4-yl) thiomethyl, [2,3-cyclopenteno-1-(2-hydroxyethyl) pyridinium-4-yl) thiomethyl, (2,3-cyclopenteno-1-dimethylaminoethylpyridinium-4-yl) thiomethyl, (2,3-cyclopenteno-1-cyclopropylpyridinium-4-yl) thiomethyl, (2,3-cyclopenteno-1-cyclopropylmethylpyridinium-4-yl) thiomethyl, (2,3-cyclopenteno-1-cyanomethylpyridinium-4-yl)thiomethyl, (5,6-cyclopenteno-1-methylpyridinium-2-yl) thiomethyl, (5,6-cyclopenteno-1-carboxyethylpyridinium-2-yl)-thiomethyl, [5,6-cyclopenteno-1-(2-hydroxyethyl) pyridinium-2-yl]thiomethyl, (2,3-cyclohexeno-1-methylpyridinium-4-yl)thiomethyl, (2,3-cyclohexeno-1-carboxyethylpyridinium-4-yl) thiomethyl, (2,3-cyclohexeno-1-carbamoylmethylpyridinium-4-yl)thiomethyl,[2,3-cyclohexeno-1-(2-hydroxyethyl) pyridinium-4-yl]thiomethyl, [2,3-cyclohexeno-1-(dimethylaminoethyl)pyridinium-4-yl) thiomethyl, pyridiniummethyl, 4-methylpyridiniummethyl, 2,3-dimethylpyridiniummethyl, 2,3-cyclopentenopyridiniummethyl, 2,3-cyclohex-enopyridiniummethyl, 4-carbamoylpyridiniummethyl, 3-carbamoylpyridiniummethyl, 4-methylthiopyridiniummethyl, 3-methylthiopyridiniummethyl, 2-methylthiopyridiniummethyl, 4-ethylthiopyridiniummethyl, 4-allyl-thiopyridiniummethyl, 4-cyclopropylmethylthiopyridiniummethyl, 3-cyclopropylmethylthiopyridiniummethyl, 4-cyclopropylthiopyridiniummethyl, 4-cyclopentylthiopyridiniummethyl, 4-(2,2,2-trifluoroethyl)-thiopyridiniummethyl, 4-(2-hydroxyethyl)thiopyridiniummethyl, 3-(2-hydroxyethyl)thiopyridiniummethyl, 4-(2-fluoroethyl)thiopyridiniummethyl, 4-carboxyethylthiopyridiniummethyl, 4-carbamoylethylthiopyridinium-methyl, 4-(N,N-dimethylaminoethyl)thiopyridiniummethyl, 2,3-cyclopenteno-4-methylthiopyridiniummethyl, 2,3-cyclopenteno-4-ethylthiopyridiniummethyl, 2,3-cyclopenteno-4-allylthiopyridiniummethyl, 2,3-cyclopenteno-4-cyclopropylmethylthiopyridiniummethyl, 2,3-cyclopenteno-2-cyclopropylthiopyridiniummethyl, 2,3-cyclopenteno-4-pentylthiopyridiniummethyl, 2,3-cyclopenteno-4-(2,2,2-trifluoroethyl) thiopyridiniummethyl, 2,3-cyclopenteno-4-(2-hydroxyethyl) thiopyridiniummethyl, 2,3-cyclopenteno-4-(2-fluoroethyl) thiopyridiniummethyl, 2,3-cyclopenteno-4-carboxymethylthiopyridiniummethyl, 2,3-cyclopenteno-4-carbamoylmethylthiopyridiniummethyl, 2, 3-cyclopenteno-4-(N,N-dimethylaminoethyl) thiopyridiniummethyl, 2,3-cyclohexeno-4-methylthiopyridiniummethyl, 2,3-cyclohexeno-4-cyclopropylmethylthiopyridiniummethyl, 2,3-cyclohexeno-4-(2,2,2-trifluoroethyl) thiopyridiniummethyl, (1H-tetrazol-5-yl)thiomethyl, (1-methyl-1H-tetrazol-5-yl) thiomethyl, (1-amino-1H-tetrazol-5-yl)thiomethyl, [1-(2-dimethylaminoethyl)-1H-tetrazol-5-yl]-thiomethyl, [1-(2-hydroxyethyl)-1H-tetrazol-5-yl]thiomethyl, [1-(2-carboxyethyl)-1H-tetrazol-5-yl]thiomethyl, (1-carboxymethyl-1H-tetrazol-5-yl)thiomethyl, (1-carbamoylmethyl-1H-tetrazol-5-yl)thiomethyl, (1-sulfomethyl-1H-tetrazol-5-yl)thiomethyl, [1-(2-sulfoethyl)-1H-tetrazol-5-yl)thiomethyl, (1-sulfamoyl-methyl-1H-tetrazol-5-yl)thiomethyl, (1,3,4-thiadiazol-5-yl)thiomethyl, (2-methyl-1,3,4-thiadiazol-5-yl)thiomethyl, (2-trifluoromethyl-1,3,4-thiadiazol-5-yl)thiomethyl, (2-carboxy-1,3,4-thiadiazol-5-yl)thiomethyl, (2-carboxymethyl-1,3,4-thiadiazol-5-yl) thiomethyl, (2-methylamino-1,3,4-thiadiazol-5-yl) thiomethyl, (2-amino-1,3,4-thiadiazol-5-yl)thiomethyl, (2-mercapto-1,3,4-thiadiazol-5-yl)thiomethyl, (2-carbamoylmethyl-1,3,4-thiadiazol-5-yl)-thiomethyl, (1,2,3-thiadiazol-5-yl)thiomethyl, (1,2,4-thiadiazol-5-yl)thiomethyl,(3-methyl-1,2,4-thiadiazol-5-yl) thiomethyl, (3-phenyl-1,2,4-thiadiazol-5-yl)thiomethyl, (thiazol-2-yl)thiomethyl, (4-methylthiazol-2-yl) thiomethyl, (4-phenylthiazol-2-yl)thiomethyl, (4-trifluoromethylthiazol-2-yl)thiomethyl, (4-carboxymethylthiazol-2-yl)thiomethyl, (5-methylthiazol-2-yl)thiomethyl, (5-phenylthiazol-2-yl) thiomethyl, (4-carboxy-3-hydroxy-isothiazol-5-yl)thiomethyl, (4-cyano-3-hydroxyisothiazol-5-yl)thiomethyl, (1,3,4-oxadiazol-5-yl)thiomethyl, (2-methyl-1,3,4-oxadiazol-5-yl)thiomethyl, (2- phenyl-1,3,4-oxadiazol-5-yl)thiomethyl, (2-carboxymethyl-1,3,4-oxadiazol-5-yl)thiomethyl, (1,2,4-oxadiazol-5-yl)thiomethyl, (3-methyl-1,2,4-oxadiazol-5-yl)thiomethyl, (3-phenyl-1,3,4-oxadiazol-5-yl)thiomethyl, (pyrazol-5-yl)thiomethyl, (1-methylimidazol-2-yl)-thiomethy], (1H- 1,2,3-triazol-5-yl)thiomethyl, (1-methyl-1H-1,2,3-triazol-5-yl) thiomethyl, (1H-1,2,4-triazol-5-yl)thiomethyl, (1-methyl-1H-1,2,4-triazol-5-yl)thiomethyl, (4-methyl-3-trifluoromethyl-4H-1,2,4-triazol-5-yl)-thiomethyl, (1H-1,3,4-triazol-5-yl)thiomethyl, (1-methyl-1H-1,3,4-triazol-5-yl) thiomethyl, (1-carboxymethyl-1H-1,3,4-triazol-5-yl) thiomethyl, (1-carbamoylmethyl-1H-1,3,4-triazol-5-yl) thiomethyl, (2-methyl-1H-1,3,4-triazol-5-yl)thiomethyl, (2-carboxymethyl-1H-1,3,4-triazol-5-yl)thiomethyl, (2-phenyl-1H-1,3,4-triazol-5-yl)-thiomethyl, (2,5-dihydro-2-methyl-5-oxo-6-hydroxy-1,2,4-triazin-3-yl)thiomethyl, (4,5-dihydro-4-methyl-5-oxo-6-hydroxy-1,2,4-triazin-3-yl) thiomethyl, (pyridazin-3-yl)thiomethyl, (2-oxypyridazin-3-yl)thiomethyl, (pyrimidin-2-yl)thiomethyl, (benzothiazol-2-yl)thiomethyl, (benzimidazol-2-yl)thiomethyl, (benzoxazol-2-yl)-thiomethyl, (3H-4-quinazolinon-2-yl)thiomethyl, (5-methyl-s-triazolo[1,5-a]pyrimi-din-7-yl)thiomethyl.

The synthesis of the above-mentioned 3-substituents may be carried out in accordance with a known method, as described in "Heterocyclic Compounds, Pyridine and its Derivatives", Vol. 14, Parts I through IV (publisehd by Interscience Publishers Ltd.). In particular, the synthesis of cyclo-ring-containing compounds such as cycloalkanothiopyridones and substituted alkylthiocycloalkanopyridines was carried out in the present invention, in accordance with the method as described in the applicant's own Japanese Patent

Application Nos. 33747/84, 138206/84 and 254518/84.

For the synthesis of the 1,5-dihydroxy-4-pyridone-2-carboxylic acid, which is a constitutional element of the 7-subsituent in the compounds of the formula (I), or protected compounds thereof; (a) a protected 5-hydroxy-4-pyrone-2- carboxylic acid is reacted with a hydroxylamino acid salt in the presence of pyridine or the like compound, as follows:

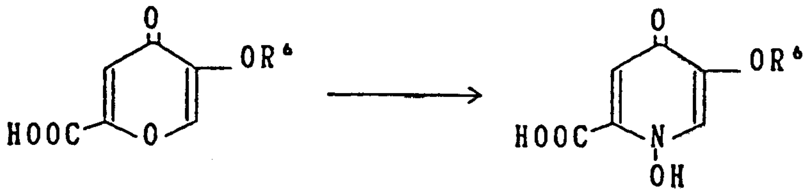

In these formulae, $R^6$ represents a removable protective group such as benzyl, p-nitrobenzyl, o-nitrobenzyl, p-methoxybenzyl or phenacyl group.

Alternatively, (b) a halogen-substituted derivative of 5-hydroxy-4-pyridone-2-carboxylic acid is oxidized with hydrogen peroxide or the like to obtain the corresponding N-oxide, which is thereafter hydrolyzed, as follows:

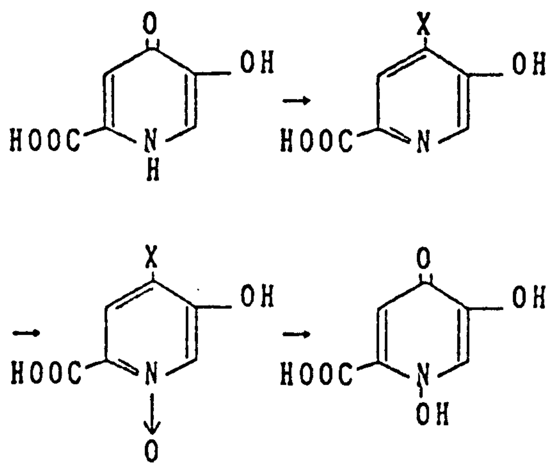

(wherein X represents a halogen atom)

The cephalosporin compound of the general formula (I) of the present invention may be prepared, according to the following method of (A), (B) or (C):

(A) A compound of a general formula (II) or a salt or silylated compound thereof:

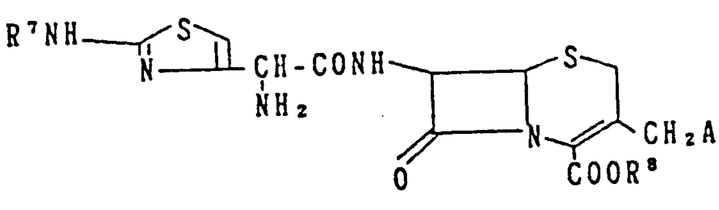

(II)

(where $R^7$ represents a hydrogen atom or an amino-protective group; $R^8$ represents a hydrogen atom or a carboxylprotective group; and A has the same meaning as above) is reacted with a compound of a general formula (III) or a carboxyl-reactive derivative thereof:

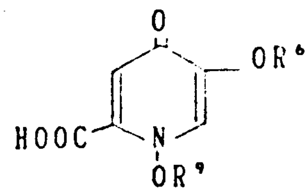

(III)

(where $R^6$ and $R^9$ may be same or different and each represents a hydrogen atom or a removable protective group such as benzyl, p-nitrobenzyl, o-nitrobenzyl, p-methoxybenzyl, methoxyethyoxymethyl or phenacyl), and then, if necessary, the protective group(s) is(are) removed, to obtain the compounds of the formula (I).

(B) A compound of a general formula (IV):

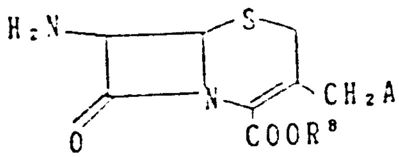

(IV)

where $R^8$ and A have the same meanings as above) is reacted with a compound of a general formula (V) or a carboxylreactive derivative thereof:

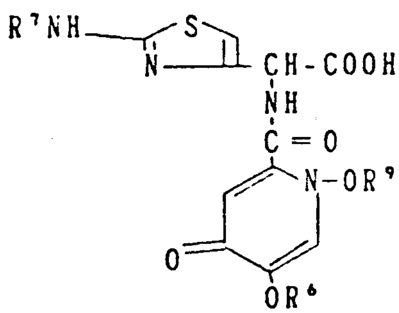

(V)

(where $R^6$, $R^7$ and $R^9$ have the same meanings as above), and then, if necessary, the protective group(s) is(are) removed, to obtain the compounds of the formula (I).

(C) A compound of a general formula (VI):

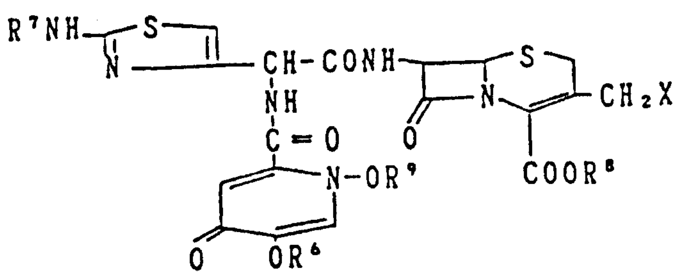

(VI)

(where $R^6$, $R^7$, $R^8$ and $R^9$ have the same meanings as above; and X represents an acetoxy group or a halogen atom) is reacted with a compound selected from formulas (VII) through (X):

a general formula

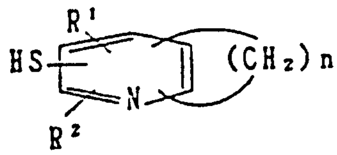

(VII)

a general formula

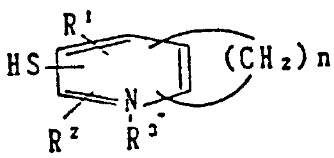

(VIII)

a general formula

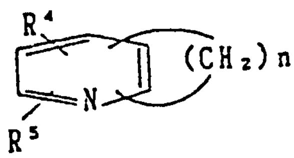

(IX)

a general formula

-S-Het (X)

(where n, $R^1$, $R^2$, $R^3$, $R^4$, $R^5$ and Het have the same meanings as above), and then, if necessary, the protective group(s) is(are) removed to obtain the compounds of the formula (I).

Regarding the amino-protective group and the carboxylprotective group in the above formulae, any conventional groups which are generally used in the field of synthesis of $\beta$-lactams and peptides for this purpose of protection of amino or carboxyl group may appropriately be adapted to the present processes (A) through (C).

Examples of amino-protective groups include phthaloyl, formyl, monochloroacetyl, dichloroacetyl, trichloroacetyl, methoxycarbonyl, ethoxycarbonyl, t-butoxycarbonyl, trichloroethoxycarbonyl, benzyloxycarbonyl, p-nitrobenzyloxycarbonyl, diphenylmethyloxycarbonyl, methoxymethyloxycarobnyl, trityl and trimethylsilyl groups; and examples of carboxylprotective groups are t-butyl, t-amyl, benzyl, p-nitrobenzyl, p-methoxybenzyl, benzhydryl, phenyl, p-nitrophenyl, methoxymethyl, ethoxymethyl, benzyloxymethyl, acetoxymethyl, methylthiomethyl, trityl, trichloroethyl, trimethylsilyl, dimethylsilyl and dimethylaminoethyl groups.

In the methods (A) and (B), the basic reaction is a condensation reaction by acylation, and general means for the acylation of penicillins and cephalosporins are adapted thereto.

Reactive derivatives which may be used in these methods include, for exmaple, acid halides, acid anhydrides, active amides and active esters. Preferred examples thereof are acid chlorides; acid bromides; mixed acid anhydrides with acetic acid, pivalic acid, isovaleric acid or trichloroacetic acid; active amides with pyrazole, imidazole, dimethylpyraozle or benzotriazole; active esters with p-nitrophenyl ester, 2, 4-dinitrophenyl ester, trichlorophenyl ester, 1-hydroxy-1H-2-pyridone, N-hydroxysuccinimide or N-hydroxyphthalimide.

In these methods, if the compound of formula (III) or (V) is used in the form of a free acid, the reaction is preferably carried out in the presence of a condensing agent; and examples of usable condensing agents are carbodiimide compounds such as N,N-dicyclohexylcarbodiimide, N-cyclohexyl-N'-morpholinoethylcarbodiimide, N-cyclohexyl-N'-(4-diethylaminocyclohexyl)carbodiimide, and reagents obtained by the reaction of an amide compound (such as N-methylformamide or N,N-dimethylformamide) and a halide (such as thionyl chloride, phosphorus oxychloride or phosgene), the reagents being known as so-called Vilsmeier reagents.

Among the reactive derivatives to be used in the present reaction, acid halides and acid anhydrides indispensably require an acid-binding agent, when reacted; and examples of the acid-binding agents usable

in the present reaction with the acid halide or acid anhydride are organic bases such as triethylamine, trimethylamine, ethyldiisopropylamine, N,N-dimethylaniline, N-methylmorpholine and pyridine; alkali metal or alkaline earth metal compounds such as sodium, potassium or calcium hydroxide, carbonate or bicarbonate; and oxiranes such as ethyleneoxide and propyleneoxide.

The present reaction is generally carried out in a solvent which does not have any inconvenient influence on the reaction; and examples of usable solvents are water, acetone, acetonitrile, dioxane, tetrahydofuran, methylene chloride, chloroform, dichloroethane, N,N-dimethylformamide or a mixture thereof.

The reaciton temperature is not specifically limitative and is generally -30°C to 40°C; and the reaction period up to the completion of the reaction is 30 minutes to 10 hours.

In case the acylated products thus obtained have any protective group(s), removal of the protective group(s) is required. For the removal of the protective group, various methods may selectively be carried out in accordance with the kind of the protective group to be removed, including, for example, a method with an acid, a method with a base or a method with a hydrazine. Conventional means which are generally utilized in the filed of synthesis of $\beta$-lactams and peptides may selectively be adapted to the present removal reaction.

The compounds of the general formula (II), which are an intermediate in the method (A), may be synthesized, according to the method as described in "J. Antibiotics 35, 1022 (1980)".

In the method (C), the reaction of the compound of the formula (VI) with the compound of the formula (VII), (VIII) or (IX) may be carried out in accordance with a conventional process which is generally carried out in the chemical field of cephalosporin compounds. More precisely, in case X in the formula (VI) is an acetoxy group, the reaction is preferably carried out, in general, in a polar solvent such as water, phosphate buffer, acetone, acetonitrile, N,N-dimethylformamide, N, N-dimethylacetamide, tetrahydrofuran, dimethylsulfoxide, dioxane, methanol or ethanol or in a mixture of the solvent and water. The reaction is preferably carried out nearly under neutral condition or so; and the reaction temperature is, though not specifically limitative, preferably from room temperature to about 70°C or so.

The time required for the completion of the present invention varies, depending upon the reaction condition, and is in general 1~10 hours. The present reaction may be accelerated, when carried out in the presence of an alkali metal halide such as sodium iodide or potassium iodide.

In case the compounds of the present invention are to be obtained from the compound of the formula (VI) where X is a halogen, the halogen may be chlorine, bromine or iodine.

The reaction is generally carried out in a solvent such as acetone, dioxane, tetrahydrofuran, ethyl acetate, acetonitrile, N,N-dimethylformamide or N,N-dimethylacetamide, and preferably under water-free condition. The reaction temperature is, in general, preferably 0~50°C, and the reaction finishes in 1~5 hours.

The compound of the formula (I) thus obtained as above may be isolated from the reaction mixture in a conventional manner.

For instance, the isolation of the compound of the formula (I) may be carried out by appropriate combinaiton of purification, sedimentation and crystallization with an adsorptive resin such as Amberlite™ XAD-2 (by Rohm and Haas Co.) or Diaion™ HP-20 (by Mitsubishi Chemical Industries, Ltd.).

The bactericides containing the compound of the formula (I) or a salt thereof as a main component may be utilized in the form of various preparations, for example, injections such as those for venoclysis or intramuscular application, peroral medicines such as capsules, tablets or granules, or per-rectal medicines, oily and fatty suppositories, water-soluble suppositories or the like various shaped medicines. These preparations may be manufactured in a conventional manner, using conventional vehicle, extender, binder, moistener, disintegrator, surfactant, lubricant, dispersing agent, buffer, preservative, solubilization-assistant, antiseptic, flavour, pain-removing agent, etc. Concrete examples for the manufacture of the present bactericidal preparations are given hereinafter in detail.

The dose of the preparation is properly determined case by case, under the consideration of the condition, age and sex of patients; and in general, the proper dose is 250~3000mg/day/adult, and this is administered to the patient as divided into 1~4 times a day.

The compounds of the formula (I) and salts thereof of the present invention are new, and have high and broad antibacterial activity capable of inhibiting the growth of pathogenic microorganisms of a broad range including gram- positive and gram-negative bacteria. In order to show the usability of the compounds of the formula (I), the antibacterial activity of some typical compounds of the formula (I) was actually measured, and the results are given in the following Table-1.

The cephalosporin derivatives of the present invention, having the 7-positioned 2-(2-aminothiazol-4-yl)-2-(1,5-dihydroxy-4-pyridone-2-carboxamido)acetamido substituent, show a broad antibacterial activity because of the synergestic effect of the 7-positioned substituent and the 3-positioned substituent; and in particular, these are especially highly active against Pseudomonas aeruginosa, and have high water-solubility which is an imporant element for injections.

The acute toxicity of the compound of the formula (I) was measured by using mouse and the $LD_{50}$ value was more than 1g/kg.

Table 1 - MIC in agar plate culture-dilution assay ($\gamma$/ml)

| Test organisms | Compound of Example 4 | Compound of Example 5 | Compound of Example 6 | Compound of Example 8 | Compound of Example 14 | Compound of Example 15 | Compound of Example 16 | Compound of Example 17 | Compound of Example 22 | Compound of Example 25 | Compound of Example 26 | A | B |
|---|---|---|---|---|---|---|---|---|---|---|---|---|---|
| Staphylococcus aureus 209P JC-1 | 3.13 | 1.56 | 1.56 | 1.56 | 1.56 | 1.56 | 3.13 | 3.13 | 3.13 | 3.13 | 3.13 | 0.78 | 3.13 |
| Staphylococcus aureus Smith(1) | 3.13 | 3.13 | 3.13 | 1.56 | 1.56 | 3.13 | 3.13 | 3.13 | 3.13 | 6.25 | 3.13 | 0.78 | 3.13 |
| Bacillus subtilis ATCC 6633 | 12.5 | 3.13 | 3.13 | 3.13 | 6.25 | 3.13 | 6.25 | 3.13 | 3.13 | 3.13 | 3.13 | 1.56 | 6.25 |
| Escherichia coli NIHJ JC-2 | 0.10 | 0.05 | 0.05 | 0.05 | 0.05 | 0.10 | 0.10 | <0.025 | 0.05 | 0.05 | 0.10 | 0.10 | 0.20 |
| Klebsiella pneumoniae GN-69 | 0.10 | 0.05 | 0.10 | 0.05 | <0.025 | 0.05 | 0.20 | 0.10 | 0.05 | 0.05 | 0.05 | 0.20 | 0.10 |
| Proteus vulgaris GN-76 | 0.39 | 0.39 | 0.20 | 0.20 | 0.10 | 0.10 | 0.39 | 0.20 | 0.10 | 0.10 | 0.20 | 0.78 | 0.05 |
| Proteus rettgeri GN-624 | 0.20 | 0.10 | 0.20 | 0.10 | 0.10 | 0.20 | 1.56 | 0.10 | 0.20 | 3.13 | 3.13 | 1.56 | 0.39 |
| Citrobacter freundii GN-346 | 6.25 | 0.78 | 1.56 | 0.78 | 0.10 | 0.20 | 0.78 | 0.78 | 0.39 | 0.78 | 0.39 | 0.39 | 0.78 |
| Enterobacter cloacae GN-7471 | 3.13 | 1.56 | 1.56 | 1.56 | 0.78 | 0.78 | 1.56 | 0.78 | 0.78 | 6.25 | 1.56 | 3.13 | 3.13 |
| Serratia marcescens No. 1 | 0.20 | 0.20 | 0.20 | 0.20 | 0.10 | 0.39 | 0.39 | 0.20 | 0.20 | 0.20 | 0.78 | 0.20 | <0.025 |
| Pseudomonas aeruginosa M-0148 | 0.39 | 0.78 | 0.78 | 1.56 | 0.20 | 0.20 | 3.13 | 1.56 | 0.39 | 0.20 | 0.20 | 50 | 3.13 |
| Pseudomonas aeruginosa E-2 | 0.10 | 0.05 | 0.05 | 0.10 | <0.025 | <0.025 | 0.10 | 0.10 | 0.10 | 0.05 | 0.05 | 0.20 | 1.56 |
| Pseudomonas aeruginosa IAM-1007 | 0.20 | 0.10 | 0.10 | 0.20 | <0.025 | 0.20 | <0.025 | 0.05 | 0.39 | 0.10 | 0.20 | 6.25 | 1.56 |
| Pseudomonas maltophilia M-0627 | - | - | - | - | 6.25 | 0.78 | 6.25 | 6.25 | 1.56 | 1.56 | 3.13 | 50 | 50 |

A; (6R,7R)7-[(RS)2-(2-aminothiazol-4-yl)-2-(5-hydroxy-4-pyridone-2-carboxamido)acetamido]-3-(1-metylpyridinium-4-yl) thiomethyl-ceph-3-em-4-carboxylate

B; ceftazidime

The present invention will be explained in greater detail by reference to the following examples.

In the Examples and Referential Examples, NMR data were based upon 100MHz- or 400MHz-NMR, and unless otherwise specifically indicated, δ-value in a deuterium oxide was an index as based upon the δ-value (=4.82) of the peak of water and δ-value in the other deuterated solvent was one as based upon the standard of TMS.

Referential Example 1: 1,5-Dihydroxy-4-pyridone-2-carboxylic acid

(a) 3.45g of sodium was added to 200ml of anhydrous methanol, to prepare a sodium methoxide solution. At room temperature, 21.3g of kojic acid was added thereto and then 19ml of benzyl chloride was added dropwise thereto, and thereafter the whole was heated under reflux and reacted for 4 hours.

After the reaction, the reaction solution was concentrated, 200ml of water was added to the remaining residue and the precipitated crystal was taken out by filtration and washed with water and ether and then dried to obtain 29g of 5-benzyloxy-2-hydroxymethyl-4-pyrone.

16g of the product was dissolved in 600ml of methanol, and 100g of active manganese dioxide was added thereto and reacted for 1 hour while heated under reflux. After the reaction, the insoluble part was filtrated out, and the remaining filtrate solution was concentrated under reduced pressure to about 200ml. To this were added 200ml of water, 69ml of 1N-NaOH and 15.9g of silver oxide, and reacted for 30 minutes at room temperature. After the reaction, the insoluble part was filtrated out, and the remaining filtrate was concentrated under reduced pressure to remove methanol therefrom and then washed with dichloromethane, and 77.5ml of 1N-HCl was added thereto to form a precipitate, which was taken out by filtration, washed with water and dried, to obtain 13g of 5-benzyloxy-4-pyrone-2-carboxylic acid.

NMR (DMSO-$d_6$) δ: 4.99(2H,s), 7.41(5H,m), 6.92(1H,s), 8.35(1H,s)

(b) 4.92g of 5-benzyloxy-4-pyrone-2-carboxylic acid was dissolved in 70ml of pyridine, and 7g of hydroxylamine hydrochloride was added thereto and reacted for 2 hours at 80 ° C. After the reaction, the reaction solution was concentrated under reduced pressure, 250ml of water was added to the remaining residue, and the resulting solution was regulated to have a pH value of 1. 5~2.0 with 6N-HCl while cooled with ice, and then this was stirred for 30 minutes at the same temperature. The precipitate formed was taken out by filtration, washed with water and dried to obtain 2.5g of 5-benzyloxy-1-hydroxy-4-pyridone-2-carboxylic acid.

NMR (DMSO-$d_6$) δ: 5.26(2H,s), 7.35-7.55(5H,m), 7.57(1H,s), 8.55(1H,s)

(c) 2g of 5-benzyloxy-1-hydroxy-4-pyridone-2-carboxylic acid was suspended in 100ml of 50%-methanol/water, 1.53ml of 1N-NaOH was added thereto and dissolved, and 500mg of 5%-palladium/carbon was added thereto and subjected to catalytic hydrogenation in a hydrogen-gas stream atmosphere at room temperature. After the reaction, 5%-palladium/carbon was filtrated out, and the remaining residue was washed with 50%-methanol/water and concentrated under reduced pressure to remove methanol therefrom, and 8ml of 1N-HCl was added thereto. The precipitate formed was taken out by filtration, washed with water and dried to obtain 1.2g of the above entitled compound.

NMR (DMSO-$d_6$) δ 7.55(1H,s), 8.05(1H,s)

Referential Example 2: 5-p-Methoxybenzyloxy-1-hydroxy-4- pyridone-2-carboxylic acid

(a) 34.5g of 5-benzyloxy-4-pyrone-2-carboxylic acid was suspended in 500ml of concentrated hydrochloric acid and 250ml of water and reacted for 1 hour at 80 ° C. The reaction solution was cooled with ice, and the formed crystal was taken out by filtration, washed with water and then dried, to obtain 16.6g of 5-hydroxy-4-pyrone-2-carboxylic acid.

NRM (DMSO-d1) δ 6.96(1H,s), 8.17(1H,s)

(b) 39.3g of 5-hydroxy-4-pyrone-2-carboxylic acid was dissolved in 700ml of DMF, and 62.5g of potassium-t-butoxide was added thereto, while cooled with ice. After 30 minutes, 75ml of p-methoxybenzyl chloride was added to the mixture and stirred for 30 minutes at room temperature and then reacted for 24 hours at 60 ° C.

After the reaction, the reaction solution was added to 2.5 lit. of dichloromethane, washed with water (800mlx3), dried with magnesium sulfate and then concentrated under reduced pressure to a little amount. Afterwards, 350ml of ether was added to the resulting residue and the crystal as precipitated was taken out by filtration, washed with ether and dried to obtain 75g of p-methoxybenzyl 5-p-methoxybenzyloxy-4-pyrone-2-carboxylate.

NMR($CDCl_3$) δ: 3.80(3H,S), 3.82(3H,S), 5.02(2H,S) 5.29(2H,s), 6.88(2H,d), 6.92(2H,d), 7.17(1H,s), 7.30(2H,d), 7.34(2H,d), 7.60(1H,s).

(c) 70g of the product as above obtained was dissolved in 1.87 lit. of tetrahydrofuran, and 476ml of water and 264ml of 1N-NaOH were added thereto and reacted for 30 minutes at room temperature. Tetrahydrofuran was removed off from the reaction mixture under reduced pressure, and then 600ml of water was added to the remaining residue and washed with dichloromethane. Next, 265ml of 1N-HCl was added thereto, while cooled with ice, and the precipitate as formed was taken out by filtration, washed with water and dried to obtain 34.9g of 5-p-methoxybenzyloxy-4-pyrone-2-carboxylic acid.

NMR(DMSO-$d_6$) δ: 3.77(3H,s), 4.90(2H,s), 6.92(1H,s), 6.96(2H,d), 7.37(2H,d), 8.34(1H,s)

(d) 33.2g of 5-p-methoxybenzyloxy-4-pyrone-2-carboxylic acid was dissolved in 500ml of pyridine, and 41.7g of hydroxylamine hydrochloride was added thereto and reacted for 1 hour at 80° C. The reaction solution was concentrated under reduced pressure to a little amount, and 800ml of water was added to the remaining residue, and the pH value thereof was adjusted to 2 with 6N-HCl, while cooled with ice. The precipitate as formed was taken out by filtration, washed with water and dried to obtain 17.25g of the above-entitled product.

NMR(DMSO-$d_6$) δ: 3.80(3H,s), 5.22(2H,s), 7.00(2H,d), 7.43(2H,d), 7.59(1H,s), 8.59(1H,s)

Referential Exmaple 3: 1-Cyclopropyl-4-thiopyridone

(a) 4.5g of pyran-4-one was dissolved in 100ml of benzene, and 10.6g of Lawesson's reagent (2,4-bis(4-methoxyphenyl)-1,3-dithia-2,4-diphosphethane-2,4-disulfide) was added thereto and stirred for 30 minutes at 80° C. After cooled, the insoluble part was filtrated out, and the remaining filtrate solution was concentrated and then subjected to silicagel-column chromatography (250g, toluene/ethyl acetate = 5/1) to obtain 5.18g (98%) of pyrane- 4-thione.

NMR($CDCl_3$) δ: 7.14(2H,d), 7.48(2H,d).

(b) 1.12g of pyran-4-thione was dissolved in 20ml of ethanol, and 1.05ml of cyclopropylamine was added thereto, while cooled with ice, and reacted for 15 minutes at room temperature. After the reaction, the reaction solution was concentrated and the remaining residue was subjected to silicagel-column chromatography (chloroform/methanol = 20/1) to obtain 1.32g of a crystal, which is the above-entitled compound.

NMR($CDCl_3$) δ: 1.08(2H,m), 1,17(2H,m), 3.49(1H,m), 7.27(2H,d), 7,38(2H,d)

Referential Example 4: 1-(2-Hydroxyethyl)-4-thiopyridone

In the same manner as the Referential Example 3(b), with the exception that ethanolamine was used, the above-entitled compound was obtained.

NMR($D_2O$) δ: 4.12(2H,m), 4,42(2H,m), 7.69(2H,d) 7.95(2H,d).

Referential Example 5: 1-(2-Dimethylaminoethyl)-4-thio pyridone

In the same manner as the Referential Example 3(b), with the exception that N,N-dimethylethylenediamine was used, the above-entitled compound was obtained.

NMR($CDCl_3$) δ: 2.24(6H,s), 2.65(2H,t), 3.96(2H,t), 7.25(2H,d), 7.42(2H,d).

Referential Example 6: 1-(2-Fluoroethyl)-4-thiopyridone

1.12g of pyran-4-thione was dissolved in 20ml of ethanol and 10ml of pyridine, and 2g of 2-fluoroethylamine hydrochloride was added thereto and reacted for 3 hours at 60° C. The reaction solution was concentrated, and then chloroform was added thereto and the insoluble part was filtrated out. The remaining filtrate solution was concentrated to a small amount and purified by means of silicagel-column chromatography (chloroform/methanol = 20/1), to obtain 890mg of the above-entitled compound.

NMR($CDCl_3$) δ: 4.17(2H,tt), 4.73(2H,tt), 7.18(2H,d) 7.42(2H,d).

Referential Exmaple 7: 1-(2-Sulfoethyl)-4-thiopyridone

1.12g of pyran-4-thione was dissolved in 20ml of ethanol, and 1.25g of 2-aminoethanesulfonic acid and 10ml of 1N-NaOH were added thereto and reacted for 1.5 hours at 70° C. After the reaction, ethanol was removed off, and the remaining reaction solution was purified with HP-20 column-chromatography to obtain 2.1g of the above-entitled compound (in the form of sodium salt).

NMR($D_2O$) δ: 3.46(2H,t), 4.57(2H,t), 7.56(2H,d), 7.88(2H,d).

Referential Example 8:1-(2-Sulfamoylethyl)-4-thiopyridone

1.12g of pyrane-4-thione was dissolved in 10ml of ethanol, and a solution obtained by dissolving 2.48g of 2-sulfamoylethylamine in a mixture comprising 10ml of ethanol and 5ml of water was added dropwise thereto. These were reacted at room temperature for 1 hour and then at 50~60°C for about 1.5 hours. After the reaction, the reaction solution was concentrated to dryness, and the residue formed was washed with methylene chloride. 30ml of water was added to the residue and the crystal as precipitated was taken out by filtration, while cooled, and thereafter washed with water and then with ether and dried to obtain 490mg of the above-entitled compound.

NMR(DMSO-$d_6$) δ: 3.52(2H,t), 4.35(2H,t), 7.08(2H,br.s), 7.14(2H,d), 7.63(2H,d).

Referential Example 9: 1-Carboxymethyl-4-thiopyridone

(a) 14.25g of 4-pyridone was dissovled in 250ml of DMF, and 25ml of ethyl bromoacetate and 31.1g of potassium carbonate were added thereto and reacted for 2. 5 hours at 60°C. After the reaction, the insoluble part was filtrated out, and the remaining filtrate solution was concentrated. The formed residue was purified with silicagel-column chromatography (chloroform/methanol = 10/1~5/1), to obtain 22.8g of 1-ethoxycarbonylmethyl-4-pyridone. This was dissolved in 400ml of dimethoxyethane and 30g of Lawesson's reagent was added thereto and reacted for 30 minutes, while heated under reflux. After the reaciton, the reaction solution was concentrated, and the residue formed was purified with silicagel-column chromatography (chloroform/methanol = 20/1) and then crystallized with dichloromethane/ether, to obtain 13.6g of 1-ethoxycarbonylmethyl-4-thiopyridone.

NMR($CDCl_3$) δ: 1.32(3H,t), 4.29(2H,q), 4.65(2H,s), 7.13(2H,d), 7.37(2H,d).

(b) 9.6g of 1-ethoxycarobnylmethyl-4-thiopyridone was dissolved in 120ml of ethanol, and 68ml of 1N-NaOH was added thereto and reacted for 20 minutes at room temperature. After the reaction, the reaction solution was concentrated to remove ethanol therefrom, and 68ml of 1N HCl was added thereto, while cooled with ice, and the precipitate formed was taken out by filtration and dried to obtain 5.23g of the above-entitled compound.

NMR(DMSO-$d_6$) δ: 4.86(2H,s), 7.18(2H,d), 7.55(2H,d)

Referential Example 10: 1-Carbamoylmethyl-4-thiopyridone

1.57g of 1-ethoxycarbonylmethyl-4-thiopyridone was added to 15ml of concentrated aqueous ammonia, while cooled with ice, and reacted for 1 hour. The reaction solution was concentrated to drying under reduced pressure, and water was added thereto and the precipitate formed was taken out by filtration. After washed with water and dried, 980mg of the above-entitled compound was obtained.

NMR(DMSO-$d_6$) δ: 4.70(2H,s), 7.16(2H,d), 7.42(1H,s), 7.50(2H,d), 7.84(1H,s).

Referential Example 11: 1-Hydroxyaminocarbonylmethyl-4-thio pyridone

200mg of hydroxylamine wad dissovled in 15ml of ethnaol, and 1.25g of 1-ethoxycarbonylmethyl-4-thiopyridone was added thereto and reacted for 24 hours at room temperature. After cooled with ice, the precipitate formed was taken out by filtration, washed with ethanol and dried to obtain 660mg of the above-entitled compound.

NMR(DMSO-$d_6$) δ: 4.62(2H,s), 7.18(2H,d), 7.50(2H,d)

Referential Exmaple 12: 1-(2-Hydroxyethyl)-3-mercapto pyridinium chloride

5.3g of 3-benzoylthiopyridine was dissolved in 30ml of acetone, and 9.4ml of 2-iodoethanol was added thereto and reacted for 16 hours, while heated under reflux. After the reaction, the reaction solution was concentrated and the remaining residue was washed with ether and then with a mixture solution of methylene chloride/ether ( = 2/1) and dried to obtain 5.3g of 1-hydroxyethyl-3-benzoylthiopyridinium iodide.

2.58g of the product as obtained above was dissolved in 35ml of 6N-HCl and reacted for 1 hour, while heated under reflux. After the reaction, the reaction solution was concentrated to drying, and the solid formed was dissolved in 20ml of water and purified with IR-120 (H+, 40ml) column-chromatography, to obtain 1.17g of the above-entitiled compound from the eluate with 3N-HCl.

NMR($D_2O$) δ: 4.03(2H,t), 4.63(2H,t), 7.87(1H,dd), 8.43(1H,d), 8.57(1H,d), 8.79(1H,s).

Referential Example 13:2,3-Dihydro-5(1H)-indolidinethione

720mg of 2,3-dihydro-6,7-bis(trimethylsilyl)-5(1H)-indolidinone (as described in "Journal of Organic Chemistry", vol. 49, p. 4786, in 1984) was stirred in 85ml of 1M tetrabutylammonium fluoride solution in THF, for 30 minutes at 60 °C. A little amount of water was added thereto and the whole was concentrated under reduced pressure, and then, after water was added thereto, this was extracted with ether. The ether layer as extracted was washed with water and dehydrated with magnesium sulfate and ether was distilled out. The residue obtained was subjected to column-chromatography with $SiO_2$ (30g) and to elution with ethyl acetate, to obtain 640mg of 2,3-dihydro-6-trimethylsilyl-5(1H)-indolidinone. Next, this was dissolved in 5ml of benzene and 5ml of trifluoroacetic acid was added thereto and stirred for 1 hour at 60 °C. The reaction solution was concentrated and ethyl acetate was added thereto, and then extracted with $NaHCO_3$-aqueous solution and water. The separated aqueous layer was extracted with chloroform and dehydrated with magnesium sulfate. Chloroform was distilled out and the residue was subjected to column-chromatography with $SiO_2$ (20g). After elution with chloroform/methanol (20/1), 183mg (53%) of 2,3-dihydro-5(1H)-indolidinone was obtained. 168mg of this product was dissolved in 5ml of benzene, and 311mg of Lawesson's reagent was added thereto and heated for 2 hours under reflux. After cooled, chloroform was added to dissolve the precipitate, and the resulting solution was then concentrated. The residue formed was subjected to column-chromatography with $SiO_2$ (20g, chloroform/methanol = 50/1) twice, to obtain 167mg (89%) of the above-entitled compound.

NMR($CDCl_3$) δ: 2.22(2H,tt), 3.20(2H,t), 4.58(2H,t), 6.55(1H,d), 7.16(1H,t), 7.43(1H,t)

Example 1:

(6R,7R)7-[(RS)2-(2-Aminothiazol-4-yl)-2-(1,5-dihydroxy-4-pyridone-2-carboxamido)acetamido]-3-acetoxymethyl-ceph-3-em-4-carboxylic acid

(Procedure A)

(a) 780mg of 5-benzyloxy-1-hdyroxy-4-pyridone-2-carboxylic acid was suspended in 12ml of tetrahydrofuran, and 0.54ml of triethylamine was added thereto and dissolved.

624mg of phosphorus pentachloride was added thereto at -15 °C and reacted for 1 hour. On the other hand, 920mg of (6R,7R)7-[(RS)2-(2-aminothaizol-4-yl)-2-aminoacetamido]-3-acetoxymethyl-ceph-3-em-4-carboxylic acid hdyrochloride was dissolved in 30ml of 50%-tetrahydrofuran aqueous solution, and triethylamine was added thereto, while cooled with ice, to regulate the pH value thereof to 8.

To this was added the previously formed tetrahydrofuran solution little by little, while the pH value of the reaction solution was kept to be 8~8.5 with triethylamine. After the reaction, the reaction solution was regulated to have pH of 6.5, and then concentrated under reduced pressure to remove tetrahydrofuran therefrom. Afterwards, water was added to the remaining residue and the resulting solution was regulated to have pH of 2 with 1N-HCl while cooled with ice. The precipitate formed was taken out by filtration and dried to obtain 1.25g of a crude powder of (6R,7R)7-[(RS)2-(2-aminothiazol-4-yl)-2-(5-benzyloxy-1-hydroxy-4-pyridone-2-carboxamido)acetamido]-3-acetoxymethyl-ceph-3-em-4-carboxylic acid. This was suspended in 20ml of 50%-methanol and then dissolved with saturated aqueous $NaHC0_3$, while the pH of the solution was regulated to be 6.5~7.0 and thereafter purified with LH-20 column-chromatography (50%-methanol/water) to obtain 500mg of sodium (6R,7R)7-[(RS)2-(2-aminothiazol-4-yl)-2-(5-benzyloxy-1-hydroxy-4-pyridone-2-carboxamido)acetamido)-3-acetoxymethyl-ceph-3-em-4-carboxylate.

(b) Next, the above obtained product was added to 30ml of aqueous water containing 500mg of palladium-black, and acetic acid was added thereto to adjust the pH value thereof to 5, and was subjected to catalytic hydrogenation at room temperature under atmospheric pressure. After the reduction, the palladium-black was filtrated out and the reaction solution was concentrated to a small amount and then was regulated to have pH of 7. 0. This was purified with HP-20 column-chromatography (for elution with $H_2O$ to 5% acetone/$H_2O$) and 250mg of the above-entitled compound was obtained in the form of sodium salt.

NMR ($D_2O$) δ 2.11, 2.12 (each 3/2H,s), 3.48(1/2x2H,ABq), 3.55(1/2x2H,ABq), 4.79(1/2x2H,ABq), 4.82(1/2x2H,ABq), 5.11(1/2H,d), 5.15(1/2H,d), 5.60(1/2H,s), 5.62(1/2H,s), 5.65(1/2H,d), 5.75(1/2H,d), 6.75(1/2H,s), 6.80(1/2H,s), 7.48(1H,s), 7.60(1H,s)

(Procedure B)

(a) 7.1g of 5-p-methoxybenzyloxy-1-hydroxy-4-pyridone-2-carboxylic acid was suspended in 100ml of tetrahydrofuran, and 4.4ml of triethylamine was added thereto and dissolved. Next, 5.33g of phosphorus pentachloride was added thereto, as divided into four times, at -10 to -15°C, and reacted at the same temperature for 1 hour, to obtain an acid chloride solution.

On the other hand, 11.9g of soidum syn-7-[2-(2-aminothiazol-4-yl)-2-methoxyiminoacetamido]-3-acetoxymethyl-ceph-3-em-4-carboxylate was dissolved in 250ml of formic acid and 20ml of water, and 11g of zinc powder was added thereto little by little, while cooled with ice. After reacted for 30 minutes, the zinc powder was filtrated out, and the remaining filtrate was, after washed with formic acid, concentrated under reduced pressure. 250ml of water was added to the residue, and hydrogen sulfide gas was introduced thereinto for 10 minutes, while cooled with ice, and the precipitate formed was filtrated out. The remaining filtrate solution was concentrated under reduced pressure and then water was added thereto to make 200ml in all. Next, 100ml of tetrahydrofuran was added thereto to obtain (6R,7R)-7-[(RS)2-(2-aminothiazol-4-yl)-2-aminoacetamido]-3-acetoxymethyl-ceph-3-em-4-carboxylic acid solution.

Triethylamine was added to the obtained solution, while cooled with ice, and the previously prepared acid chloride solution was added thereto, while the pH value of the solution was kept to be 8-8.5, and reacted for 1 hour.

After the reaction, tetrahydrofuran was removed off under reduced pressure, and the pH value of the solution was made to be 2 with 6N-HCl, while cooled with ice, and thus, the precipitate formed was taken out by filtration, washed with water and dried to obtain 9.2g of a crude powder of (6R,7R)7-[(RS)2-(2-aminothiazol-4-yl)-2-(5-p-methoxybenzyloxy-1-hydroxy-4-pyridone-2-carboxamido) acetamido]-3-acetoxymethyl- ceph-3-em-4-carboxylic acid.

(b) The product obtained above was suspended in 22ml of anisole, and 97ml of trifluoroacetic acid was added dropwise thereto, while cooled with ice, and reacted for 30 minutes at room temperature. Afterwards, the reaction mixture was dropped into 700ml of diisopropylether.

The precipitate formed was taken out by filtration, washed with diisopropylether and dried. The precipitate obtained was dissolved in about 60ml of saturated sodium hdyrogencarbonate aqueous solution (pH 7.2~7.4) and purified with HP-20 column-chromatography, to obtain 3. 34g of the above entitled compound in the form of soidum salt. The spectral data of the compound obtained were same as those of the same compound obtained by the Method A in the above.

(Procedure C)

171mg of 1,5-dihydroxy-4-pyridone-2-carboxylic acid was suspended in 5ml of tetrahydrofuran, and 0.18ml of triethylamine and 208mg of phosphorus pentachloride were added tehreto, while cooled with ice, and reacted for 1 hour at room temperature. On the other hand, 300mg of (6R,7R)7-[(RS)2-(2-aminothiazol-4-yl)-2-aminoacetamido]-3-acetoxymethyl-ceph-3-em-4-carboxylic acid hydrochloride was dissolved in 10ml of 50%-tetrahydrofuran aqueous solution, and the solution was regulated to have pH of 7. 5 with saturated $NaHC0_3$ aqueous solution. To this was added the previously prepared tetrahydrofuran solution little by little, while this was cooled with ice and the pH of the reaction solution was regulated to be 8.0. After the reaction, the pH of the reaction solution was regulated to be 6.0, and the solution was concentrated under reduced pressure to remove tetrahydrofuran therefrom. The remaining aqueous solution was regulated to have pH of 2.0 with 1N-HCl, and the precipitate formed was taken out by filtration and dried, and then this was purified with HP-20 and LH-20 column chromatography, in the same manner as mentioned above, to obtain 60mg of sodium salt of the above-entitled compound. The spectral data of the compound obtained were same as those of the same compound obtained by the Method A in the above.

Example 2: (6R,7R)7-[(RS)2-(2-Aminothiazol-4-yl)-2-(1,5-dihydroxy-4-pyridone-2-carboxamido)acetamido]-3-(1-methylpyridinium-4-yl)thiomethyl-ceph-3-em-4-carboxylate

960mg of sodium (6R,7R)7-[(RS)2-(2-aminothiazol-4-yl)-2-(1,5-dihydroxy-4-pyridone-2-carboxamido)-acetamido]-3-acetoxymethyl-ceph-3-em-4-carboxylate (as obtained in the above Example 1) was dissolved in 8ml of acetonitrile and 8ml of water, and 2.5g of sodium iodide and 360mg of 1-methyl-4-thiopyridone were added thereto and, after the pH of the reaction solution was adjusted to 6.8, reacted for 4.5 hours at 65~70°C. After the reaction, the reaction solution was added dropwise to 200ml of acetone, and the precipitate formed was taken out by filtration. After washed with acetone and dried, the obtained precipitate was dissolved in 15ml of water and then purified with HP-20 column chromatography (development solvent: 5% acetone aqueous solution), to obtain 380mg of the above-entitled product. This was further purified with LH-20 column chromatography (50% methanol aqueous solution), to obtain 230mg of the product.

NMR($D_2O$) δ: 3.54(1/2x2H,ABq), 3.58(1/2x2H,ABq), 4.29(1/2x2H,ABq), 4.32(1/2x2H,ABq), 4.22(3H,s), 5.09(1/2H,d), 5.13(1/2H,d), 5.62(1H,s), 5.62(1/2H,d), 5.73(1/2H,d), 6.76(1/2H,s), 6.81-(1/2H,s), 7.39(1/2H,s), 7.40(1/2H,s), 7.63(1/2H,s), 7.64(1/2H,s), 7.81(2H,d), 8.40(2H,d).

In the same manner as the Example 2, with the exception that the reagent of (A) as given below was used instead of 1-methyl-4-thiopyridone, products of the following Examples 3 through 39 were obtained.

Example 3:

(6R,7R)7-[(RS)2-(2-Aminothiazol-4-yl)-2-(1,5-dihydroxy-4-pyridone-2-carboxamido)acetamido]-3-(1-carboxymethylpyridinium-4-yl)thiomethyl-ceph-3-em-4-carboxylate

(A): 1-Carboxymethyl-4-thiopyridone

NMR($D_2O$) δ: 3.38(1/2H,d), 3.43(1/2H,d), 3.66(1/2H,d), 3.70(1/2H,d), 4.15(1/2H,d), 4.17(1/2H,d), 4.40-(1/2H,d), 4.43(1/2H,d), 5.06(2H,s), 5.08(1/2H,d), 5.11(1/2H,d), 5.62(1H,s), 5.63(1/2H,d), 5.73(1/2H,d), 6.74(1/2H,s), 6.78(1/2H,s), 7.40(1H,s), 7.66(1H,s), 7.81(2H,d), 8.37(2H,d).

Example 4:

(6R,7R)7-[(RS)2-(2-Aminothiazol-4-yl)-2-(1,5-dihydroxy-4-pyridone-2-carboxamido)acetamido]-3-(1-methylpyridinium-3-yl)thiomethyl-ceph-3-em-4-carboxylate

(A): 3-Mercapto-1-methylpyridinium chloride

NMR($D_2O$) δ: 3.38(1/2H,d), 3.43(1/2H,d), 3.62(1/2H,d), 3.64(1/2H,d), 3.96(1/2H,d), 3.99(1/2H,d), 4.19-(1H,d), 4.30(3/2H,s), 4.31(3/2H,s), 5.01(1/2H,d), 5.04(1/2H,d), 5.51(1/2H,d), 5.58(1/2H,s), 5.59(1/2H,s), 5.60(1/2H,d), 6.69(1/2H,s), 6.74(1/2H,s), 7.33(1H,s), 7.57(1/2H,s), 7.58-(1/2H,s), 7.85(1H,m), 8.37(1H,m), 8.57(1H,d), 8.85(1H,s).

Example 5:

(6R,7R)7-[(RS)2-(2-Aminothiazol-4-yl)-2-(1,5-dihydroxy-4-pyridone-2-carboxamido)acetamido]-3-[1-(2-hydoroxyethyl)pyridinium-4-yl]thiomethyl-ceph-3-em-4-carboxylate

(A) 1-(2-Hydroxyethyl)-4-thiopyridone

NMR($D_2O$) δ : 3.37(1/2H,d), 3.43(1/2H,d), 3.63(1/2H,d), 3.68(1/2H,d), 4.01(2H,t), 4.14(1/2H,d), 4.17-(1/2H,d), 4.37(1/2H,d), 4.40(1/2H,d), 4.53(2H,m), 5.07(1/2H,d), 5.11(1/2H,d), 5.60(1H,s), 5.61(1/2H,d), 5.70(1/2H,d), 6.73(1/2H,s), 6.77(1/2H,s), 7.29(1/2H,s), 7.30(1/2H,s), 7.52-(1/2H,s), 7.54(1/2H,s), 7.79(2H,m), 8.43(2H,m)

Example 6:

(6R,7R)7-[(RS)2-(2-Aminothiazol-4-yl)-2-(1,5-dihydroxy-4-pyridone-2-carboxamido)acetamido]-3-(1-carbamoylmethylpyridinium-4-yl)thiomethyl-ceph-3-em-4-carboxylate

(A); 1-Carbamoylmethyl-4-thiopyridone

NMR($D_2O$) δ: 3.40(1/2H,d), 3.45(1/2H,d), 3.65(1/2H,d), 3.71(1/2H,d), 4.17(1/2H,d), 4.22(1/2H,d), 4.41-(1/2H,d), 4.45(1/2H,d), 5.08(1/2H,d), 5.11(1/2H,d), 5.33(2H,br.s.), 5.61(1/2H,d), 5.61-(1H,s), 5.71(1/2H,d), 6.74(1/2H,s), 6.79(1/2H,s), 7.37(1H,s), 7.59(1/2H,s), 7.60(1/2H,s), 7.85(2H,m), 8.40(2H,m).

Example 7:

(6R,7R)7-[(RS)2-(2-Aminothiazol-4-yl)-2-(1,5-dihydroxy-4-pyridone-2-carboxamido)acetoamido)-3-[1-(2-dimethylaminoethyl)pyridinium-4-yl]thiomethyl-ceph-3-em-4-carboxylate

(A): 1-(2-Dimethylaminoethyl)-4-thiopyridone

NMR($D_2O$) δ: 2.47(3H,s), 2.50(3H,s), 3.20(2H,m), 3.37(1/2H,d), 3.41(1/2H,d), 3.64(1/2H,d), 3.68-(1/2H,d), 4.14(1H,d), 4.38(1H,d), 4.65(2H,m), 5.08(1/2H,d), 5.12(1/2H,d), 5.60(1/2H,d), 5.61(1H,s), 5.71(1/2H,d), 6.72(1/2H,s), 6.77(1/2H,s), 7.31(1/2H,s), 7.35(1/2H,s), 7.54-(1/2H,s), 7.58(1/2H,s), 7.82(2H,m), 8.47(2H,m)

Example 8:

(6R,7R)7-[(RS)2-(2-Aminothiazol-4-yl)-2-(1,5-dihydroxy-4-pyridone-2-carboxamido)acetamido]-3-(1-cyclopropylpyridinium-4-yl)thiomethyl-ceph-3-em-4-carboxylate

(A): 1-Cyclopropyl-4-thiopyridone

NMR($D_2O$) δ: 1.30(4H,m), 3.36(1/2H,d), 3.42(1/2H,d), 3.63(1/2H,d), 3.67(1/2H,d), 4.15(2H,m), 4.38-(1/2H,d), 4.42(1/2H,d), 5.05(1/2H,d), 5.09(1/2H,d), 5.57(1H,s), 5.58(1/2H,d), 5.68(1/2H,d), 6.73(1/2H,s), 6.77(1/2H,s), 7.37(1/2H,s), 7.38(1/2H,s), 7.59(1/2H,s), 7.60(1/2H,s), 7.76-(2H,m), 8.52(2H,d).

Example 9:

(6R,7R)7-[(RS)2-(2-Aminothiazol-4-yl)-2-(1,5-dihydroxy-4-pyridone-2-carboxamido)acetamido]-3-(1-hydroxyaminocarbonylmethylpyridinium-4-yl)thiomethyl-ceph-3-em-4-carboxylate

(A): 1-Hydroxyaminocarbonylmethyl-4-thiopyridone

NMR($D_2O$) δ: 3.41(1/2H,d), 3.46(1/2H,d), 3.69(1/2H,d), 3.73(1/2H,d), 4.17(1/2H,d), 4.20(1/2H,d), 4.43-(1/2H,d), 4.47(1/2H,d), 5.06(2H,s), 5.10(1/2H,d), 5.14(1/2H,d), 5.61(1H,s), 5.61(1/2H,d), 5.73(1/2H,d), 6.76(1/2H,s), 6.80(1/2H,s), 7.43(1H,s), 7.68(1H.s), 7.83(2H,m), 8.39(2H,d)

Example 10:

(6R,7R)7-[(RS)2-(2-Aminothiazol-4-yl)-2-(1,5-dihydroxy-4-pyridone-2-carboxamido)acetamido]-3-[1-(2-fluoroethyl) pyridinium-4-yl] thiomethyl-ceph-3-em-4-carboxylate

(A): 1-(2-Fluoroethyl)-4-thiopyridone

NMR($D_2O$) δ: 3.37(1/2H,d), 3.43(1/2H,d), 3.65(1/2H,d), 3.68(1/2H,d), 4.13(1/2H,d), 4.17(1/2H,d), 4.42-(1/2H,d), 4.45(1/2H,d), 4.7~4.95(4H,m), 5.05(1/2H,d), 5.10(1/2H,d), 5.57(1H,s), 5.58-(1/2H,d), 5.68(1/2H,d), 6.73(1/2H,s), 6.78(1/2H,s), 7.38(1H,s), 7.62(1H,s), 7.87(2H,m), 8.48(2H,d)

Example 11:

(6R,7R)7-[(RS)2-(2-Aminothiazol-4-yl)-2-(1,5-dihydroxy-4-pyridone-2-carboxamido)acetamido]-3-[1-(2-hydroxyethyl) pyridinium-3-yl]thiomethyl-ceph-3-em-4-carboxylate

(A): 1-(2-Hydroxyethyl)-3-mercaptopyridinium chloride

NMR($D_2O$) δ: 3.40(1/2H,d), 3.47(1/2H,d), 3.70(1/2H,d), 3.71(1/2H,d), 4.03(2H,m), 4.10(2H,ABq), 4.65-(2H,m), 5.04(1/2H,d), 5.07(1/2H,d), 5.50(1/2H,d), 5.58(1H,s), 5.61(1/2H,d), 6.71(1/2H,s), 6.76(1/2H,s), 7.37(1/2H,s), 7.38(1/2H,s), 7.60(1H,s), 7.94(1H,m), 8.50(1H,m),8.68(1H,m), 8.99(1H,br.s.).

Example 12:

(6R,7R)7-[(RS)2-(2-Aminothiazol-4-yl)-2-(1,5-dihydorxy-4-pyridone-2-carboxamido)acetamido]-3-[1-(2-sulfoethyl) pyridinium-4-yl]thiomethyl-ceph-3-em-4-carboxylate

(A): 1-(2-Sulfoethyl)-4-thiopyridone

NMR($D_2O$) δ: 3.36(1/2H,d), 3.42(1/2H,d), 3.54(2H,t), 3.64(1/2H,d), 3.68(1/2H,d), 4.13(1/2H,d), 4.15-(1/2H,d), 4.37(1/2H,d), 4.41(1/2H,d), 4.82(2H,m), 5.07(1/2H,d), 5.10(1/2H,d), 5.58(1/2H,s), 5.59(1/2H,s), 5.61(1/2H,d), 5.70(1/2H,d), 6.73(1/2H,s), 6.77(1/2H,s), 7.38(1/2H,s), 7.39-(1/2H,s), 7.62(1/2H,s), 7.63(1/2H,s), 7.78(1H,d), 7.80(1H,d), 8.49(1H,d), 8.51(1H,d).

Example 13:

(6R,7R)7-[(RS)2-(2-Aminothiazol-4-yl)-2-(1,5-dihydroxy-4-pyridone-2-carboxamido)acetamido]-3-[1-(2-sulfamoylethyl) pyridinium-4-yl]thiomethyl-ceph-3-em-4-carboxylate

(A): 1-(2-Sulfamoylethyl)-4-thiopyridone

NMR($D_2O$) δ: 3.35(1/2H,d), 3.39(1/2H,d), 3.60(1/2H,d), 3.67(1/2H,d), 3.93(2H,t), 4.17(1/2H,d), 4.18-(1/2H,d), 4.34(1/2H,d), 4.38(1/2H,d), 4.92(2H,br.s), 5.06(1/2H,d), 5.09(1/2H,d), 5.59-(1/2H,s), 5.59(1/2H,s), 5.60(1/2H,d), 5.69(1/2H,d), 6.71(1/2H,s), 6.75(1/2H,s), 7.35-(1/2H,s), 7.36(1/2H,s), 7.59(1/2H,s), 7.60(1/2H,s), 7.80(1H,d), 7.81(1H,d), 8.51(2H,d).

Example 14:

(6R,7R)7-[(RS)2-(2-Aminothiazol-4-yl)-2-(1,5-dihydroxy-4-pyridone-2-carboxamido)acetamido]-3-(2,3-cyclopenteno-1-methylpyridinium-4-yl)-thiomethyl-ceph-3-em-4-carboxylate

(A): 1-Methylcyclopentano[b]4-thiopyridone

NMR($D_2O$) δ: 2.28(2H,m), 3.88(2H,m), 3.21(2H,m), 3.55(1/2x2H,ABq), 3.60(1/2x2H,ABq), 4.04(3H,s), 4.26(1/2x2H, ABq), 4.28(1/2x2H, ABq), 5.09(1/2H,d), 5.13(1/2H,d), 5.62(1/2H,d), 5.63-(1H,s), 5.72(1/2H,d), 6.75(1/2H,s), 6.79(1/2H,s), 7.26(1/2H,s), 7.31(1/2H,s), 7.53(1H,d), 7.54((1/2H,s), 7.57(1/2H,s), 8.15(1H,d)

Example 15:

(6R,7R)7-[(RS)2-(2-Aminothiazol-4-yl)-2-(1,5-dihydroxy-4-pyridone-2-carboxamido)acetamido]-3-(2,3-cyclopentenopyridine-4-yl)thiomethyl-ceph-3-em-4-carboxylic acid

(A): Cyclopentano[b]4-thiopyridone

NMR($D_2O$) δ: 2.16(2H,m), 2.82(2H,m), 3.01(2H,m), 3.49(1/2x2H,ABq), 3.55(1/2x2H,ABq), 4.17-(1/2x2H,ABq), 4.20(1/2x2H,ABq), 5.02(1/2H,d), 5.07(1/2H,d), 5.60(1/2H,d), 5.62(1H,s), 5.70(1/2H,d), 6.75(1/2H,s), 6.80(1/2H,s), 7.21(1H,d), 7.37(1/2H,s), 7.39(1/2H,s), 7.61-(1/2H,s), 7.62(1/2H,s), 8.12(1H,d)

Example 16:

(6R,7R)7-[(RS)2-(2-Aminotniazol-4-yl)-2-(1,5-dihydroxy-4-pyridone-2-carboxamido)acetamido]-3-(pyridine-4-yl) thiomethyl-ceph-3-em-4-carboxylic acid

(A): 4-Mercaptopyridine

NMR($D_2O$) δ: 3.50(1/2x2H,ABq), 3.57(1/2x2H,ABq), 4.15(1/2x2H,ABq), 4.17(1/2x2H,ABq), 5.01(1/2H,d), 5.05(1/2H,d), 5.58(1/2H,d), 5.60(1H,s), 5.67(1/2H,d), 6.74(1/2H,s), 6.79(1/2H,s), 7.40-(2H,d), 7.42(1H,s), 7.62(1H,s), 8.33(2H,d)

Example 17:

(6R,7R)7-[(RS)2-(2-Aminothiazol-4-yl)-2-(1,5-dihydroxy-4-pyridone-2-carboxamido)acetamido]-3-(2,3-cyclopenteno-1-carboxylmethylpyridinium-4-yl)-thiomethyl-ceph-3-em-4-carboxylate

(A): 1-Carboxymethyl-cyclopentano[b]4-thiopyridone

NMR($D_2O$) δ: 2.30(2H,m), 2.98(2H,m), 3.17(2H,m), 3.53(1/2x2H,ABq), 3.58(1/2x2H,ABq), 4.30-(1/2x2H,ABq), 4.32(1/2x2H,ABq), 4.94(2H,s), 5.06(1/2H,d), 5.10(1/2H,d), 5.60(1H,s), 5.61-(1/2H,d), 5.71(1/2H,d), 6.75(1/2H,s), 6.79(1/2H,s), 7.40(1/2H,s), 7.41(1/2H,s), 7.60(1H,m), 7.65(1/2H,s), 7.66(1/2H,s), 8.18(1H,m)

Example 18:

(6R,7R)7-[(RS)2-(2-Aminothiazol-4-yl)-2-(1,5-dihydroxy-4-pyridone-2-carboxamido)acetamido]-3-(2,3-cyclopentenopyridinium)methyl-ceph-3-em-4-carboxylate

(A): 2,3-Cyclopentenopyridine

NMR($D_2O$) δ: 2.33(2H,m), 3.20(3H,m), 3.35(2H,m), 3.45(1/2H,d), 3.48(1/2H,d), 5.15(1/2H,d), 5.18-(1/2H,d), 5.32(1H,m), 5.48(1H,m), 5.61(1H,s), 5.68(1/2H,d), 5.78(1/2H,d), 6.75(1/2H,s), 6.79(1/2H,s), 7.43(1H,s), 7.69(1/2H,s), 7.70(1/2H,s), 7.80(1H,m), 8.30(1H,m), 8.53-(1/2H,d), 8.56(1/2H,d)

Example 19:

(6R,7R)7-[(RS)2-(2-Aminothiazol-4-yl)-2-(1,5-dihydroxy-4-pyridone-2-carboxamido)acetamido]-3-(2,3-cyclopenteno-4-methylthiopyridinium)methyl-ceph-3-em-4-carboxylate

(A): 2,3-Cyclopenteno-4-methylthiopyridine

NMR($D_2O$) δ: 2.33(2H,m), 2.68(3/2H,s), 2.69(3/2H,s), 2.95(2H,m), 3.27(2H,m), 3.15 - 3.50(2H,m), 5.15-(1/2H,d), 5.18(1/2H,d), 5.10~5.35(2H,m), 5.60(1H,s), 5.67(1/2H,d), 5.78(1/2H,d), 6.75-(1/2H,s), 6.79(1/2H,s), 7.37(1H,s), 7.50(1/2H,d), 7.52(1/2H,d), 7.62(1H,s), 8.32(1/2H,d), 8.34(1/2H,d).

Example 20:

(6R,7R)7-[(RS)2-(2-Aminothiazol-4-yl)-2-(1,5-dihydroxy-4-pyridone-2-carboxamido)acetamido]-3-(4-methylthiopyridinium)methyl-ceph-3-em-4-carboxylate

(A): 4-Methylthiopyridine

NMR($D_2O$) δ: 2.62(3/2H,s), 2.64(3/2H,s), 3.35(1/2x2H,ABq), 3.40(1/2x2H,ABq), 5.10(1H,m), 5.18-(1/2H,d), 5.20(1/2H,d), 5.36(1H,m), 5.57(1/2H,s), 5.58(1/2H,s), 5.67(1/2H,d), 5.78(1/2H,d), 6.70(1/2H,s), 6.74(1/2H,s), 7.33(1H,s), 7.60(1H,s), 7.70(1H,d), 7.74(1H,d), 8.53(2H,m)

Example 21

(6R,7R)7-[(RS)2-(2-Aminothiazol-4-yl)-2-(1,5-dihydroxy-4-pyridone-2-carboxamido)acetamido]-3-(1-methyl-1H-tetrazol-5-yl)thiomethyl-ceph-3-em-4-carboxylic acid

(A): 5-Mercapto-1-methyl-1H-tetrazole

NMR($D_2O$) δ: 7.64(1H,s), 7.40(1H,s), 6.81(1/2H,s), 6.76(1/2H,s), 5.71(1/2H,d), 5.62(1/2H,d), 5.63-(1/2H,s), 5.61(1/2H,s), 5.12(1/2H,d), 5.09(1/2H,d), 4.35(1/2H,d), 4.32(1/2H,d), 4.07-(3/2H,s), 4.05(3/2H,s), 4.05(1H,m), 3.78(1/2H,d), 3.74(1/2H,d), 3.47(1/2H,d), 3.41(1/2H,d)

Example 22:

(6R,7R)7-[(RS)2-(2-Aminothaizol-4-yl)-2-(1,5-dihydroxy-4-pyridone-2-carboxamido)acetamido]-3-(4,5-dihydro-4-methyl-5-oxo-6-hydroxy-1,2,4-triazin-3-yl)thiomethyl-ceph-3-em-4-carboxylic acid

(A): 4,5-Dihydro-3-mercapto-4-methyl-5-oxo-6-hydroxy-1, 2,4- triazine

NMR($D_2O$) δ: 7.64(1H,s), 7.40(1H,s), 6.80(1/2H,s), 6.75(1/2H,s), 5.72(1/2H,d), 5.63(3/2H,m), 5.10-(1/2H,d), 5.07(1/2H,d), 4.50(1/2H,d), 4.48(1/2H,d), 3.80(2H,m), 3.47(3/2H,s), 3.45(3/2H,s), 3.41(1/2H,d), 3.35(1/2H,d).

Example 23:

(6R,7R)7-[(RS)-2-(2-Aminothiazol-4-yl)-2-(1,5-dihydroxy-4-pyridone-2-carboxamido)acetamido]-3-(1-amino-1H-tetrazol-5-yl)thiomethyl-ceph-3-em-4-carboxylic acid

(A): 5-Mercapto-1-amino-1H-tetrazole

NMR($D_2O$) δ: 7.61(1H,s), 7.38(1H,s), 6.81(1/2H,s), 6.76(1/2H,s), 5.75(1/2H,d), 5.65(1/2H,d) 5.61-(1/2H,s), 5.59(1/2H,s), 5.17(1/2H,d), 5.13(1/2H,d), 4.12(1H,s), 4.10(1H,s), 3.68(1/2H,d), 3.63(1/2H,d), 3.41(1/2H,d), 3.37(1/2H,d).

Example 24:

(6R,7R)7-[(RS)2-(2-Aminothiazol-4-yl)-2-(1,5-dihydroxy-4-pyridone-2-carboxamido)acetamido]-3-[1-(2-dimethylaminoethyl)-1H-tetrazol-5-yl]thiomethyl-ceph-3-em-4-carboxylic acid

(A): 5-Mercapto-1-(2-dimethylaminoethyl)-1H-tetrazole

NMR($D_2O$) δ: 7.63(1H,s), 7.41(1H,s), 6.81(1/2H,s), 6.75(1/2H,s), 5.69(1/2H,d), 5.61(1H,s), 5.58(1/2H,d), 5.13(1/2H,d), 5.10(1/2H,d), 4.80(2H,m), 4.28(1/2H,d), 4.27(1/2H,d), 4.13(1/2H,d), 4.10-(1/2H,d), 3.77(1/2H,d), 3.73(1/2H,d), 3.65(2H,m), 3.52(1/2H,d), 3.46(1/2H,d), 2.85(3/2H,s), 2.87(3/2H,s)

Example 25:

(6R,7R)7-[(RS)2-(2-Aminothiazol-4-yl)-2-(1,5-dihydroxy-4-pyridone-2-carboxamido)acetamido]-3-(1,2,3-thiadiazol-5-yl)thiomethyl-ceph-3-em-4-carboxylic acid

(A): 5-Mercapto-1,2,3-thiadiazole

NMR($D_2O$) δ: 8.69(1/2H,s), 8.68(1/2H,s), 7.61(1H,s), 7.37(1H,s), 6.78(1/2H,s), 6.73(1/2H,s), 5.68-(1/2H,d), 5.60(1/2H,s), 5.59(1/2H,s), 5.59(1/2H,d), 5.09(1/2H,d), 5.06((1/2H,d), 4.37-(1/2H,d), 4.32(1/2H,d), 3.92(1/2H,d), 3.90(1/2H,d), 3.71(1/2H,d), 3.67(1/2H,d), 3.40-(1/2H,d), 3.34(1/2H,d).

Example 26:

(6R,7R)7-[(RS)2-(2-Aminothiazol-4-yl)-2-(1,5-dihydroxy-4-pyridone-2-carboxamido)acetamido]-3-(benzothiazol-2-yl) thiomethyl-ceph-3-em-4-carboxylic acid

(A): 2-Mercaptobenzothiazole

NMR($D_2O$) δ: 7.81(1H,d), 7.76(1H,d), 7.55(1/2H,s), 7.53(1/2H,s), 7.46(1H,m), 7.36(1H,m), 7.31(1/2H,s), 7.27(1/2H,s), 6.73(1/2H,s), 6.70(1/2H,S), 5.66(1/2H,d), 5.56(1H,s), 5.56(1/2H,d), 5.01-(1/2H,d), 4.96(1/2H,d), 4.53(1/2H,d), 4.49(1/2H,d), 4.06(1/2H,d), 4.02(1/2H,d), 3.68-(1/2H,d), 3.63(1/2H,d), 3.38(1/2H,d), 3.30(1/2H,d)

Example 27:

(6R,7R)7-[(RS)2-(2-Aminothiazol-4-yl)-2-(1,5-dihydroxy-4-pyridone-2-carboxamido)acetamido]-3-(2,5-dihydro-2-methyl-5-oxo-6-hydroxy-1,2,4-triazin-3-yl)thiomethyl-ceph-3-em-4-carboxylic acid

(A): 3-Mercapto-2,5-dihydro-2-methyl-5-oxo-6-hydroxy-1, 2,4- triazine

NMR($D_2O$) δ: 3.50(1/2x2H,ABq), 3.58(1/2x2H,ABq), 3.65, 3.66(each1/2x3H,s), 4.21(1/2x2H,ABq), 4.24-(1/2x2H,ABq), 5.04(1H,s), 5.10(1/2H,d), 5.14(1/2H,d), 5.63(1/2H,d), 5.72(1/2H,d), 6.75-(1/2H,s), 6.80(1/2H,s), 7.35(1/2H,s), 7.36(1/2H,s), 7.58(1H,s)

Example 28:

(6R,7R)7-[(RS)2-(2-Aminothiazol-4-yl)-2-(1,5-dihydroxy-4-pyridone-2-carboxamido)acetamido]-3-(2-methyl-1,3,4-thiadiazol-5-yl)thiomethyl-ceph-3-em-4-carboxylic acid

(A): 5-Mercapto-2-methyl-1,3,4-thiadiazole

NMR($D_2O$) δ: 7.71(1H,s), 7.45(1H,s), 6.80(1/2H,s), 6.73(1/2H,s), 5.67(1/2H,d), 5.59(1/2H,s), 5.58-(1/2H,s), 5.58(1/2H,d), 5.08(1/2H,d), 5.05(1/2H,d), 4.50(1/2H,d), 4.48(1/2H,d), 3.93-(1/2H,d), 3.89(1/2H,d), 3.78(1/2H,d), 3.75(1/2H,d), 3.40(1/2H,d), 3.34(1/2H,d), 2.72(3H,s)

Example 29:

(6R,7R)7-[(RS)2-(2-Aminothiazol-4-yl)-2-(1,5-dihydroxy-4-pyridone-2-carboxamido)acetamido]-3-(2-methylamino-1,3,4-thiadiazol-5-yl)thiomethyl-ceph-3-em-4-carboxylic acid

(A): 5-Mercapto-2-methylamino-1,3,4-thiadiazole

NMR($D_2O$) δ: 2.95, 2.96(each 1/2x3H,s), 3.44(1/2x2H,ABq), 3.52(1/2x2H,ABq), 4.30(1/2x2H,ABq), 4.33-(1/2x2H,ABq), 5.04(1/2H,d), 5.10(1/2H,d), 5.60(1/2H,d), 5.61(1H,s), 5.67(1/2H,d), 6.76-(1/2H,s), 6.80(1/2H,s), 7.42(1/2H,s), 7.43(1/2H,s), 7.66(1H,s).

Example 30:

(6R,7R)7-[(RS)2-(2-Aminothiazol-4-yl)-2-(1,5-dihydroxy-4-pyridone-2-carboxamido)acetamido]-3-(benzimidazol-2-yl) thiomethyl-ceph-3-em-4-carboxylic acid

(A): 2-Mercaptobenzimidazole

NMR($D_2O$) δ: 7.65~7.1(6H,m), 6.83(1/2H,s), 6.79(1/2H,s), 5.73(1/2H,d), 5.69(1/2H,d), 5.65(1/2H,s), 5.64(1/2H,s), 5.19(1/2H,d), 5.11(1/2H,d), 4.51(1/2H,d), 4.47(1/2H,d), 3.98(1/2H,d), 3.95-(1/2H,d), 3.81(1/2H,d), 3.76(1/2H,d), 3.55(1/2H,d), 3.50(1/2H,d)

Example 31:

(6R,7R)7-[(RS)2-(2-Aminothiazol-4-yl)-2-(1,5-dihydroxy-4-pyridone-2-carboxamido)acetamido]-3-(benzoxazol-2-yl) thiomethyl-ceph-3-em-4-carboxylic acid

(A): 2-Mercaptobenzoxazole

NMR($D_2O$) δ: 7.60(3H,m), 7.37(3H, m), 6.77(1/2H,s), 6.73(1/2H,s), 5.65(1/2H,d), 5.57(1/2H,s), 5.55-(1/2H,s), 5.56(1/2H,d), 5.06(1/2H,d) 5.02(1/2H,d) 4.71(1/2H,d), 4.68(1/2H,d), 4.03(1/2H,d), 3.99(1/2H,d), 3.81(1/2H,d), 3.78(1/2H,d), 3.45(1/2H,d), 3.38(1/2H,d).

Example 32:

(6R,7R)7-[(RS)2-(2-Aminothiazol-4-yl)-2-(1,5-dihydroxy-4-pyridone-2-carboxamido)acetamido]-3-(5-methyl-s-triazolo-[1,5-a]pyrimidin-7-yl)thiomethyl-ceph-3-em-4-carboxylic acid

(A): 7-Mercapto-5-methyl-s-triazolo[1,5-a]pyrimidine

NMR($D_2O$) δ : 2.61(3H,s), 3.43(1/2H,d), 3.48(1/2H,d), 3.71(1/2H,d), 3.75(1/2H,d), 4.19(1/2H + 1/2H,m),

4.50(1/2H,d), 4.54(1/2H,d), 5.08(1/2H,d), 5.11(1/2H,d), 5.58(1/2H,d), 5.63(1/2H,d), 5.72-(1/2H,d), 6.72(1/2H,s), 6.77(1/2H,s), 7.17(1H,s), 7.32(1/2H,s), 7.33(1/2H,s), 7.57(1/2H,s), 7.59(1/2H,s), 8.45(1/2H,s), 8.46(1/2H,s)

Example 33:

(6R,7R)7-[(RS)2-(2-Aminothiazol-4-yl)-2-(1,5-dihydroxy-4-pyridone-2-carboxamido)acetamido]-3-(3-trifluoromethyl-4-methyl-4H-1,2,4-triazol-5-yl)thiomethyl-ceph-3-em-4-carboxylic acid

(A): 5-Mercapto-4-methyl-3-trifluoromethyl-4H-1,2,4-triazole

NMR($D_2O$) δ: 3.37(1/2H,d), 3.44(1/2H,d), 3.68~3.86(2H,m), 3.84(3H,s), 4.28(1/2H,d), 4.33(1/2H,d), 5.07-(1/2H,s), 5.10(1/2H,s), 5.59(1/2H,d), 5.60(1/2H,s), 5.61(1/2H,s), 5.68(1/2H,d), 6.75-(1/2H,s), 6.80(1/2H,s), 7.40(1H,s), 7.63(1H,s).

Example 34:

(6R,7R)7-[(RS)2-(2-Aminothiazol-4-yl)-2-(1,5-dihydroxy-4-pyridone-2-carboxamido)acetamido]-3-(pyrimidin-2-yl) thiomethyl-ceph-3-em-4-carboxylic acid

(A): 2-Mercaptopyrimidine

NMR($D_2O$) δ: 3.35(1/2H,d), 3.42(1/2H,d), 3.8(1/2H,m), 4.21(1H,m), 4.65(1H,m), 5.03(1/2H,d), 5.07-(1/2H,d), 5.61(1H,d), 5.63(1/2H,d), 5.69(1/2H,d), 6.75(1/2H,s), 6.81(1/2H,s), 7.22(1H,t), 7.41(1H,s), 7.68(1H,s), 8.58(2H,m)

Example 35:

(6R,7R)7-[(RS)2-(2-Aminothiazol-4-yl)-2-(1,5-dihydroxy-4-pyridone-2-carboxamido)acetamido]-3-(4-carboxy-3-hydroxyisothiazol-5-yl)thiomethyl-ceph-3-em-4-carboxylicacid

(A): 4-Carboxy-3-hydroxy-5-mercaptoisothiazole

NMR($D_2O$) δ: 3.42(1/2H,d), 3.48(1/2H,d), 3.63(1/2H,d), 3.68(1/2H,d), 4.29(2H,m), 5.12(1/2H,d), 5.17-(1/2H,d), 5.63(1/2H,d), 5.64(1H,s), 5.74(1/2H,d), 6.78(1/2H,s), 6.82(1/2H,s), 7.44(1H,s), 7.68(1H,s).

Example 36:

(6R,7R)7-[(RS)2-(2-Aminothiazol-4-yl)-2-(1,5-dihydroxy-4-pyridone-2-carboxamido)acetamido]-3-(4-cyano-3-hydroxyisothiazol-5-yl)thiomethyl-ceph-3-em-4-carboxylic acid

(A): 4-Cyano-3-hydroxy-5-mercaptoisothiazole

NMR($D_2O$) δ: 3.41(1/2H,d), 3.50(1/2H,d), 3.63(1/2H,d), 3.68(1/2H,d), 4.30(2H,m), 5.13(1/2H,d), 5.18-(1/2H,d), 5.63(1/2H,d), 5.64(1H,s), 5.75(1/2H,d), 5.78(1/2H,s), 6.82(1/2H,s), 7.44(1H,s), 7.66(1H,s).

Example 37:

(6R,7R)7-[(RS)2-(2-Aminothiazol-4-yl)-2-(1,5-dihydroxy-4-pyridone-2-carboxamido)acetamido]-3-(2-carboxy-1-methylpyridinium-4-yl)thiomethyl-ceph-3-em-4-carboxylate

(A): 2-Carboxy-1-methyl-4-thiopyridone

NMR($D_2O$) δ: 3.36(1/2H,d), 3.42(1/2H,d), 3.65(1/2H,d), 3.69(1/2H,d), 4.15(1H,m), 4.16(3H,s), 4.33-(1/2H,d), 4.37(1/2H,d), 5.07(1/2H,d), 5.10(1/2H,d), 5.57(1/2H,s), 5.59(1/2H,s), 5.60-(1/2H,d), 5.70(1/2H,d), 6.72(1/2H, s), 6.77(1/2H,s), 7.38(1H,s), 7.62(1/2H,s), 7.62(1/2H,s),

7.69(1H,m), 7.79(1H,m), 8.32(1H,m).

Example 38:

(6R,7R)7-[(RS)2-(2-Aminothiazol-4-yl)-2-(1,5-dihydroxy-4-pyridone-2-carboxamido)acetamido]-3-(2,3-dihydro-1H-indolidinium-5-yl)thiomethyl-ceph-3-em-4-carboxylate

(A): 2,3-Dihydro-5(1H)-indolidinethione

NMR($D_2O$) δ: 2.46(2H,m), 3.41(1H,m), 3.50(2H,m), 3.67(1H,m), 4.20(1H,m), 4.44(1H,m), 4.64(2H,m), 5.03(1/2H,d), 5.07(1/2H,d), 5.56(1/2H,d), 5.59(1H,s), 5.65(1/2H,d), 6.73(1/2H,s), 6.77-(1/2H,s), 7.38(1/2H,s), 7.39(1/2H,s), 7.61(1H,s), 7.63(1H,m), 7.68(1H,m), 8.13((1H,m).

Example 39:

(6R,7R)7-[(RS)2-(2-Aminothiazol-4-yl)-2-(1,5-dihydroxy-4-pyridone-2-carboxamido)acetamido]-3-(4-phenylthiazol-2-yl)thiomethyl-ceph-3-em-4-carboxylic acid

(A): 2-Mercapto-4-phenylthiazole

NMR($D_2O$) δ: 7.76((2H,d), 7.67(1H,s), 7.60(1/2H,s), 7.57(1/2H,s), 7.35 - 7.50(3H,m), 7.35(1/2H,s), 7.33-(1/2H,s), 6.71(1/2H, s), 6.68(1/2H,s), 5.63(1/2H,d), 5.54((1/2H,s), 5.53(1/2H,d), 5.00-(1/2H,d), 4.95(1/2H,d), 4.47(1/2H,d), 4.43(1/2H,d), 3.90(1/2H,d), 3.88(1/2H,d), 3.68-(1/2H,d), 3.62(1/2H,d), 3.34(1/2H,d), 3.26(1/2H,d)

Example 40:

(6R,7R]7-[(RS)2-(2-Aminothiazol-4-yl)-2-(1,5-dihydroxy-4-pyridone-2-carboxamido)acetamido]-3-(1-methyl-1H-tetrazol-5-yl)thiomethyl-ceph-3-em-4-carboxylic acid

In the same manner as the method A of the Exmaple 1, with the exception that (6R,7R)7-[(RS)2-(2-aminothiazol-4-yl)-2-aminoacetamido]-3-(1-methyl-1H-tetrazol-5-yl) thiomethyl-ceph-3-em-4-carboxylic acid hydrochloride was used instead of (6R,7R)7-[(RS)2-(2-aminothiazol-4-yl)-2-aminoacetamido]-3-acetoxymethyl-ceph-3-em-4-carboxylic acid hydrochloride, the above-entitled compound was obtained. The spectral data of this compound were same as those of the compound of the Example 21.

Example 41:

(6R,7R)7-[(RS)2-(2-Aminothiazol-4-yl)-2-(1,5-dihydroxy-4-pyridone-2-carboxamido)acetamido]-3-(1-methylpyridinium-4-yl)thiomethyl-ceph-3-em-4-carboxylate

(a) 1.03g of (RS)2-(2-tritylaminothiazol-4-yl)-2-(t-butoxycarbonylamino)acetic acid was dissolved in 10ml of DMF, and 270mg of 1-hydroxybenzotriazole and 412mg of N, N'-dicyclohexylcarbodiimide were added thereto and reacted for 1 hour at room temperature.

To this was added 10ml of DMF-solution containing 740mg of benzhydryl 7-amino-3-chloromethyl-ceph-3-em-4-carboxylate and reacted for 5 hours at room temperature. After the reaction, the insoluble part was filtrated out, and 120ml of ethyl acetate was added to the remaining filtrate solution, which was thereafter washed with dilute sodium hydrogencarbonate aqueous solution, dilute hydrochloric acid aqueous solution and saturated salt aqueous solution in this order and then dried with magnesium sulfate and concentrated to drying under reduced pressure. The residue formed was purified with silicagel-colum chromatography (benzene/ethyl acetate = 8/1), to obtain 1.3g of benzhydryl (6R,7R)7-[(RS)2-(2-aminothiazol-4-yl)-2-(t-butoxycarbonylamino)acetamido]-3-chloromethyl-ceph-3-em-4-carboxylate.

(b) 1.05g of the product as above obtained was dissolved in 15ml of tetrahydrofuran, and 180mg of 1-methyl-4-thiopyridone was added thereto and reacted for 5 hours at room temperature. After the reaction, the reaction solution was concentrated, 20ml of ethyl acetate was added to the resulting residue and the precipitate formed was taken out by filtration and then washed with ethyl acetate. After filtrated, 1g of benzhydryl (6R,7R) 7-[(RS)2-(2-tritylaminothiazol-4-yl)-2-(t-butoxycarbonylamino)acetamido]-3-(1-methylpyridinium-4-yl)thiomethyl-ceph-3-em-4-carboxylate was obtained.

(c) 980mg of the product as above obtained was suspended in 3.3ml of anisole, and 15ml of trifluoroacetic acid was added thereto, while cooled with ice, and reacted for 30 minutes at room temperature. After the reaction, the reaction solution was added dropwise to 100ml of diisopropylether, and the precipitate formed therein was taken out by filtration and dried to obtain 720mg of (6R,7R)7-[-(RS)2-(2-aminothiazol-4-yl)-2-aminoacetamido]-3-(1-methylpyridinium-4-yl)thiomethyl-ceph-3-em-4-carboxylate trifluoroacetate.

NMR($D_2O$) $\delta$: 3.49(1/2H,d), 3.56(1/2H,d), 3.75(1/2H,d), 3.77(1/2H,d), 4.22(3H,s), 4.3 - 4.5(2H,m), 5.16(1/2H,d), 5.18(1/2H,d), 5.32(1/2H,s), 5.33(1/2H,s), 5.65(1/2H,d), 5.77(1/2H,d), 7.12-(1H,s), 7.81(2H,m), 8.43(2H,d)

(d) 380mg of 5-p-methoxybenzyloxy-1-hydroxy-4-pyridone-2-carboxylic acid was suspended in 6ml of tetrahydrofuran, and 0.24ml of triethylamine was added thereto and dissolved. 220mg of phosphorus pentachloride was added thereto at -10 to -15°C and reacted for 1 hour at the same temperature, to obtain an acid chloride solution.

670mg of (6R,7R)7-[(RS)2-(2-aminothiazol-4-yl)-2-aminoacetamido]-3-(1-methylpyridinium-4-yl)-thiomethyl-ceph-3-em-4-carboxylate trifluoroacetate, as obtained in the above (c), was dissolved in 3ml of tetrahydrofuran and 9ml of water, and then the previously prepared acid chloride-solution was added thereto, while the reaction system was cooled with ice and was regulated to have pH of 8-8.5 with triethylamine. After the reaction, the reaction solution was concentrated under reduced pressure to remove tetrahydrofuran therefrom, and 6N-HCl was added thereto, while cooled with ice, to adjust the pH thereof to 2, and the precipitate formed was taken out by filtration. After washed with water and dried, 550mg of a crude powder of (6R,7R)7-[(RS)2-(2-aminothiazol-4-yl)-2-(5-p-methoxybenzyloxy-1-hydroxy-4-pyridone-2-carboxamido)acetamido]-3-(1-methylpyridinium-4-myl) thiomethyl-ceph-3-em-4-carboxylate was obtained.

(e) The product obtained in (d) was suspended in 1. 5ml of anisole and 6ml of trifluoroacetic acid was added thereto, while cooled with ice, and reacted for 30 minutes at room temperature.

After the reaction, the reaction solution was added to 60ml of diisopropylether, and the precipitate formed was taken out by filtration and dried. This was suspended in a small amount of water,and saturated sodium hydrogencarbonate aqueous solution was added thereto and dissolved (pH 7~7.2), and the resulting solution was purified with HP-20 column-chromatography (eluent: 5% acetone aqueous solution), to obtain 240mg of the above entitled compound. The spectral data of this compound were same as those of the same compound of the Example 2.

Example 42:

(6R,7R)7-[(RS)2-(2-Aminothiazol-4-yl)-2-(1,5-dihydroxy-4-pyridone-2-carboxamido)acetamido]-3-(1,2,3-thiadiazol-5-yl)thiomethyl-ceph-3-em-4-carboxylic acid

(a) 520mg of 5-benzyloxy-1-hydroxy-4-pyridone-2-carboxylic acid was suspended in 7ml of tetrahydrofuran, and 0.36ml of triethylamine was added thereto and dissolved. 420mg of phosphorus pentachloride was added thereto at -10°C and reacted for 1 hour, to obtain an acid chloride solution.

On the other hand, 600mg of (RS)2-(2-tritylaminothiazol-4-yl)glycine was dissolved in 20ml of 50%-tetrahydrofuran aqueous solution. To this was added the previously prepared acid chloride solution little by little, while the pH value of the reaction solution was kept to be 8~8.5 with triethylamine and cooled with ice.

After the reaction, the reaction solution was concentrated under reduced pressure to remove tetrahydrofuran therefrom, and 6N-HCl was added to the residue to regulate the pH of the solution to 2, which was then extracted with chloroform (100mlx2). After washed with water, the liquid was dried with magnesium sulfate and concentrated to drying to obtain 1.0g of (RS)2-(2-tritylaminothiazol-4-yl)-2-(5-benzyloxy-1-hydroxy-4-pyridone-2-carboxamido)acetic acid.

(b) 660mg of the product obtained above was dissolved in 5ml of tetrahydrofuran, and 0.15ml of triehtylamine and 210mg of phosphorus pentachloride were added thereto at 10°C and reacted for 1 hour at the same temperature.

On the other hand, 250mg of 7-amino-3-(1,2,3-thiadiazol-5-yl)thiomethyl-ceph-3-em-4-carboxylic acid was suspended in 50%-tetrahydrofuran aqueous solution, and triethylamine was added thereto, while cooled with ice, and dissolved at pH 8. To this was added the previously prepared reaction solution little by little, while the pH value of the reaction solution was kept to be 8-8.5 with triethylamine as added.

After the reaction, the reaction solution was concentrated under reduced pressure to remove tetrahydrofuran therefrom, and then, water was added thereto and the resulting solution was regulated to

have pH of 2 with 6N-HCl, which was thereafter extracted with ethyl acetate. After washed with water, the liquid was dried with magnesium sulfate and concentrated to drying, to obtain 790mg of (6R,7R)7-[(RS)2-(2-tritylaminothiazol-4-yl)-2-(5-benzyloxy-1-hydroxy-4-pyridone-2-carboxamido)acetamido]-3-(1,2,3-thiadiazol-5-yl) thiomethyl-ceph-3-em-4-carboxylic acid.

(c) This was dissolved in 7ml of formic acid, and 0. 3ml of concentrated hydrochloric acid was added thereto and reacted for 1 hour at room temperature. After the reaction, the precipitate formed was filtrated out, and the remaining filtrate solution was concentrated to a small amount and then washed with ether.

The residue obtained was suspended in a small amount of water and then dissolved, as regulated to have pH of 7 with saturated sodium hydrogencarbonate aqueous solution. The resulting solution was purified with HP-20 column-chromatography (eluent: 10-20% acetone/water) to obtain 320mg of sodium (6R,7R)7-[(RS)2-(2-aminothiazol-4-yl)-2-(5-benzyloxy-1-hydroxy-4-pyridone-2-carboxamido) acetamido]-3-(1,2,3-thiazol-5-yl)thiomethyl-ceph-3-em-4-carboxylate.

Next, this was subjected to catalytic hydrogenation in the same manner as the step (b) in the Exmaple 1, to obtain the above-entitled compound. The spectral data of this compound were same as those of the same compound of the Example 25.

Example 43: Preparations for injection

The compound of the Example 2 was filled in vials under sterile condition, each in an amount of 1000mg (titer).

Example 44: Capsules

| | |
|---|---|
| Compound of Example 2 | 250 parts (titer) |
| Lactose | 60 parts |
| Magnesium stearate | 5 parts |

These were blended uniformly, and the resulting mixture was encapsulated in an amount of 250mg (titer)/one capsule.

Example 45: Soft capsules for per-rectal application

| | |
|---|---|
| Olive oil | 160 parts |
| Polyoxyethylenelaurylether | 10 parts |
| Sodium hexametaphosphate | 5 parts |

These were blended uniformly to obtain a base component. 25 parts (titer) of the compound of the Example 2 were added to the base component and further uniformly blended and then encapsulated in soft capsules each in an amount of 250mg (titer)/one capsule, to obtain soft capsules for per-rectal application.

## Claims

1. New cephalosporin compounds represented by the following formula (I) and pharmaceutically acceptable salts thereof:

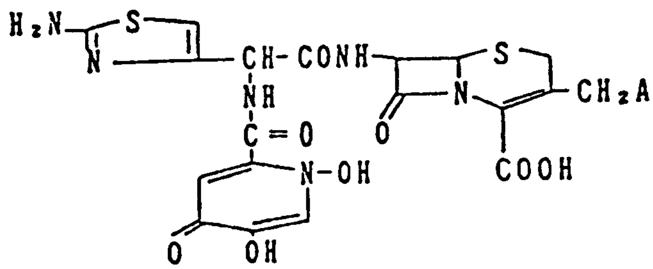

(I)

wherein A represents an alkanoyloxy group having 2-5 carbon atoms; a carbamoyloxy group; an azido group; or an unsubstituted or substituted pyridylthio group of a formula (I-1):

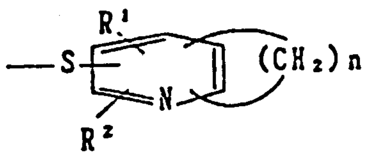

(I-1)

(where n is 0 or an integer of 3-5; $R^1$ and $R^2$ may be same or different and each represent a hydrogen atom, a halogen atom, a carboxyl group or an optionally halogen-substituted lower alkyl group having 1-5 carbon atoms); or an unsubstituted or substituted pyridiniumthio group of a formula (I-2):

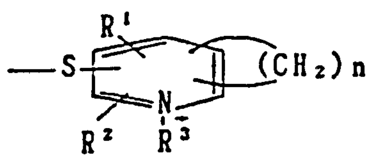

(I-2)

[where n, $R^1$ and $R^2$ have the same meanings as above; $R^3$ represents a linear or branched alkyl group having 1-5 carbon atoms, a halogen-substituted alkyl group, a cyclopropyl group, a cyclopropylmethyl group, an alkenyl group, an oxygen atom or a group of $-(CH_2)_m-B$; (m is an integer of 0-3; and B represents a hydroxyl group, an alkoxy group, an amino group, an alkyl-substituted amino group, a carboxyl group, a carbamoyl group, a sulfonic acid group, a sulfonic acid amide group, a hydroxamic acid group, a cyano group, a thiol group, an alkylthio group, a methanesulfonylaminocarbonyl group or an acetamidosulfonyl group)]; or an unsubstituted or substituted pyridinium group of a formula (I-3):

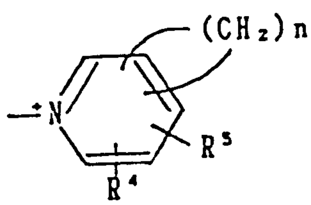

(I-3)

(where n has the same meaning as above; $R^4$ and $R^5$ may be same or different and each represent a hydrogen atom, a linear or branched alkyl group having 1~5 carbon atoms, a carboxyl group, a carbamoyl group, a sulfonic acid group, a sulfonic acid amide group, a linear or branched alkylthio group having 1~5 carbon atoms, a halogen-substituted alkylthio group, a cycloalkanothio group, a cycloalkanomethylthio group, a carboxyalkylthio group, a carbamoylalkylthio group, an alkoxyalkylthio group or an alkylsbustituted aminoalkylthio group); or a 5- or 6-membered heterocyclicthio or bicycloheterocyclicthio group of a formula (I-4):

-S-Het (I-4)

(where Het represents an optionally substituted thiazole, isothiazole, 1,2,3-thiadiazole, 1,3,4-thiadiazole, 1,3,4-triazole, 1,2,3,4-tetrazole, pyrimidine, 1,2,4-triazine, benzothiazole, benzimidazole, benzoxazole, 1,3,4-triazaindolidine or 2,3-dihydro-1H-indolidinium group).

**2.** (6R,7R)7-[(RS)2-(2-aminothiazol-4-yl)-2-(1,5-dihydroxy-4-pyridone-2-carboxamido)acetamido]-3-[1-(2-hydroxy)ethylpyridinium-4-yl]thiomethyl-ceph-3-em-4-carboxylate.

**3.** (6R,7R)7-[(RS)2-(2-aminothiazol-4-yl)-2-(1,5-dihydroxy-4-pyridone-2-carboxamido)acetamido]-3-(1-cyclopropyl-pyridinium-4-yl)thiomethyl-ceph-3-em-4-carboxylate.

**4.** (6R,7R)7-[(RS)2-(2-aminothiazol-4-yl)-2-(1,5-dihydroxy-4-pyridone-2-carboxamido)acetamido]-3-(1-methylpyridinium-4-yl)thiomethyl-ceph-3-em-4-carboxylate.

**5.** (6R,7R)7-[(RS)2-(2-aminothiazol-4-yl)-2-(1,5-dihydroxy-4-pyridone-2-carboxamido)acetamido]-3-(1-carboxymethyl-pyridinium-4-yl)thiomethyl-ceph-3-em-4-carboxylate.

**6.** (6R,7R)7-[(RS)2-(2-aminothiazol-4-yl)-2-(1,5-dihydroxy-4-pyridone-2-carboxamido)acetamido]-3-(1-carbamoylmethyl-pyridinium-4-yl)thiomethyl-ceph-3-em-4-carboxylate.

**7.** (6R,7R)7-[(RS)2-(2-aminothiazol-4-yl)-2-(1,5-dihydroxy-4-pyridone-2-carboxamido)acetamido]-3-(2,3-cyclopenteno-1-carboxymethyl-pyridinium-4-yl)thiomethyl-ceph-3-em-4-carboxyalte.

**8.** (6R,7R)7-[(RS)2-(2-aminothaizol-4-yl)-2-(1,5-dihydroxy-4-pyridone-2-carboxamido)acetamido]-3-(1-methylpyridinium-3-yl)thiomethyl-ceph-3-em-4-carboxylate.

**9.** (6R,7R)7-[(RS)2-(2-aminothiaozl-4-yl)-2-(1,5-dihydroxy-4-pyridone-2-carboxamido)acetamido]-3-[1-(2-sulfamoyl)ethylpyridinium-4-yl]thiomethyl-ceph-3-em-4-carboxylate.

**10.** (6R,7R)7-[(RS)2-(2-aminothiazol-4-yl)-2-(1,5-dihydroxy-4-pyridone-2-carboxamido)acetamido]-3-(1,2,3-thiadiazol-5-yl)thiomethyl-ceph-3-em-4-carboxylate.

**11.** (6R,7R)7-[(RS)2-(2-aminothiazol-4-yl)-2-(1,5-dihydroxy-4-pyridone-2-carboxamido)acetamido]-3-(2,3-cyclopenteno-pyridinium)methyl-ceph-3-em-4-carboxylate.

**12.** (6R,7R)7-[(RS)2-(2-aminothiazol-4-yl)-2-(1,5-dihydroxy-4-pyridone-2-carboxamido)acetamido]-3-(4-methylthiopyridinium)methyl-ceph-3-em-4-carboxylate.

**13.** (6R,7R)7-[(RS)2-(2-aminothiazol-4-yl)-2-(1,5-dihydroxy-4-pyridone-2-carboxamido)acetamido]-3-(1-methyltetrazol-5-yl)thiomethyl-ceph-3-em-4-carboxyalte.

**14.** (6R,7R)7-[(RS)2-(2-aminothiazol-4-yl)-2-(1,5-dihydroxy-4-pyridone-2-carboxamido)acetamido]-3-(benzothiazol-2-yl)thiomethyl-ceph-3-em-4-carboxylate.

**15.** (6R,7R)7-[(RS)2-(2-aminothiazol-4-yl)-2-(1,5-dihydroxy-4-pyridone-2-carboxamido)acetamido]-3-[1-(2-fluoroethyl)pyridinium-4-yl]thiomethyl-ceph-3-em-4-carboxylate.

**16.** (6R,7R)7-[(RS)2-(2-aminothiazol-4-yl)-2-(1,5-dihydroxy-4-pyridone-2-carboxamido)acetamido]-3-(pyridin-4-yl)thiomethyl-ceph-3-em-4-carboxylate.

**17.** An antibacterial pharmaceutical composition containing an effective amount of a compound of claim 1 and a pharmaceutically acceptable carrier.

**Patentansprüche**

**1.** Neue Cephalosporin-Verbindungen der folgenden Formel (I) und pharmazeutisch verträgliche Salze davon:

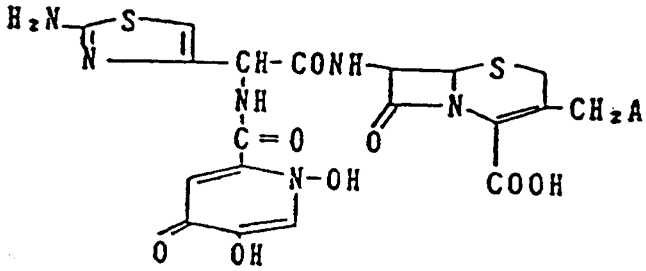

(I)

worin A einen Alkanoyloxy-Rest mit 2-5 C-Atomen, einen Carbamoyloxy-Rest, einen Azido-Rest oder einen unsubstituierten oder substituierten Pyridylthio-Rest der Formel (I-1):

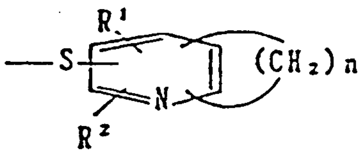

(I-1)

(worin n 0 oder eine ganze Zahl von 3-5 ist, $R^1$ und $R^2$ gleich oder verschieden voneinander sein können und jeweils ein Wasserstoffatom, ein Halogenatom, einen Carboxyl-Rest oder einen gegebenenfalls Halogen-substituierten Niederalkyl-Rest mit 1-5 C-Atomen bedeuten), oder einen unsubstituierten oder substituierten Pyridiniumthio-Rest der Formel (I-2):

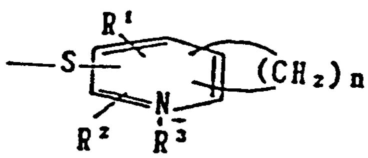

(I-2)

[worin n, $R^1$ und $R^2$ die vorstehend angegebenen Bedeutungen haben, $R^3$ einen linearen oder verzweigten Alkyl-Rest mit 1-5 C-Atomen, einen Halogen-substituierten Alkyl-Rest, einen Cyclopropyl-Rest, einen Cyclopropylmethyl-Rest, einen Alkenyl-Rest, ein Sauerstoffatom oder einen Rest von $-(CH_2)_m-B$ bedeutet, (m eine ganze Zahl von 0-3 ist, und B einen Hydroryl-Rest, einen Alkoxy-Rest, einen Amino-Rest, einen Alkyl-substituierten Amino-Rest, einen Carboxyl-Rest, einen Carbamoyl-Rest, einen Sulfonsäure-Rest, einen Sulfonsäureamid-Rest, einen Hydroxamsäure-Rest, einen Cyano-Rest, einen Thiol-Rest, einen Alkylthio-Rest, einen Methansulfonylaminocarbonyl-Rest oder einen Acetamidosulfonyl-Rest bedeutet)], oder einen unsubstituierten oder substituierten Pyridinium-Rest der Formelm (I-3):

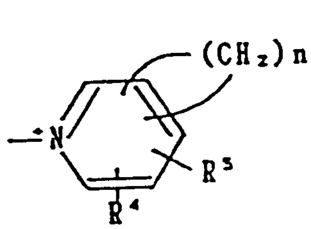

(I-3)

(worin n die vorstehend angegebene Bedeutung hat, $R^4$ und $R^5$ gleich oder verschieden voneinander sein können und jeweils ein Wasserstoffatom, einen linearen oder verzweigten Alkyl-Rest mit 1-5 C-Atomen, einen Carboryl-Rest, einen Carbamoyl-Rest, einen Sulfonsäure-Rest, einen Sulfonsäureamid-Rest, einen linearen oder verzweigten Alkylthio-Rest mit 1-5 C-Atomen, einen Halogen-substituierten Alkylthio-Rest, einen Cycloalkanothio-Rest, einen Cycloalkanomethylthio-Rest, einen Carboxyalkylthio-Rest, einen Carbamoylalkylthio-Rest, einen Alkoxyalkylthio-Rest oder einen alkylsubstituierten Aminoalkylthio-Rest bedeuten), oder einen 5- oder 6-gliedrigen heterocyclischen Thio- oder bicycloheterocyclischen Thio-Rest der Formel (I-4):

-S-Het (I-4)

bedeutet (worin Het einen gegebenenfalls substituierten Thiazol-, Isothiazol-, 1,2,3-Thiadiazol-, 1,3,4-Thiadiazol-, 1,3,4-Triazol-, 1,2,3,4-Tetrazol-, Pyrimidin-, 1,2,4-Triazin-, Benzothiazol-, Benzimidazol-, Benzoxazol-, 1,3,4-Triazaindolidin- oder 2,3-Dihydro-1H-Indolidinium-Rest bedeutet).

**2.** (6R,7R)7-[(RS)2-(2-Aminothiazol-4-yl)-2-(1,5-dihydroxy-4-pyridon-2-carboxamido)acetamido]-3-[1-(2-hydroxy)ethylpyridinium-4-yl]thiomethyl-ceph-3-em-4-carboxylat .

**3.** (6R,7R)7-[(RS)2-(2-Aminothiazol-4-yl)-2-(1,5-dihydroxy-4-pyridon-2-carboxamido)acetamido]-3-(1-cyclopropyl-pyridinium-4-yl)thiomethyl-ceph-3-em-4-carboxylat .

**4.** (6R,7R)7-[(RS)2-(2-Aminothiazol-4-yl)-2-(1,5-dihydroxy-4-pyridon- 2-carboxamido)acetamido]-3-(1-methylpyridinium-4-yl)thiomethyl-ceph-3-em-4-carboxylat.

**5.** (6R,7R)7-[(RS)2-(2-Aminothiazol-4-yl)-2-(1,5-dihydroxy-4-pyridon-2-carboxamido)acetamido]-3-(1-carboxymethyl-pyridinium-4-yl)thiomethyl-ceph-3-em-4-carboxylat.

**6.** (6R,7R)7-[(RS)2-(2-Aminothiazol-4-yl)-2-(1,5-dihydroxy-4-pyridon-2-carboxamido)acetamido]-3-(1-carbamoylmethyl-pyridinium-4-yl)thiomethyl-ceph-3-em-4-carboxylat.

**7.** (6R,7R)7-[(RS)2-(2-Aminothiazol-4-yl)-2-(1,5-dihydroxy-4-pyridon-2-carboxamido)acetamido]-3-(2,3-cyclopenteno-1-carboxymethyl-pyridinium-4-yl)thiomethyl-ceph-3-em-4-carboxylat.

**8.** (6R,7R)7-[(RS)2-(2-Aminothaizol-4-yl)-2-(1,5-dihydroxy-4-pyridon-2-carboxamido)acetamido]-3-(1-methylpyridinium-3-yl)thiomethyl-ceph-3-em-4-carboxylat.

**9.** (6R,7R)7-[(RS)2-(2-Aminothiaozl-4-yl)-2-(1,5-dihydroxy-4-pyridon-2-carboxamido)acetamido]-3-[1-(2-sulfamoyl)ethylpyridinium-4-yl]thiomethyl-ceph-3-em-4-carboxylat.

**10.** (6R,7R)7-[(RS)2-(2-Aminothiazol-4-yl)-2-(1,5-dihydroxy-4-pyridon-2-carboxamido)acetamido]-3-(1,2,3-thiadiazol-5-yl)thiomethyl-ceph-3-em-4-carboxylat .

**11.** (6R,7R)7-[(RS)2-(2-Aminothiazol-4-yl)-2-(1,5-dihydroxy-4-pyridon-2-carboxamido)acetamido]-3-(2,3-cyclopenteno-pyridinium)methyl-ceph-3-em-4-carboxylat .

**12.** (6R,7R)7-[(RS)2-(2-Aminothiazol-4-yl)-2-(1,5-dihydroxy-4-pyridon-2-carboxamido)acetamido]-3-(4-methylthiopyridinium)methyl-ceph-3-em-4-carboxylat .

**13.** (6R,7R)7-[(RS)2-(2-Aminothiazol-4-yl)-2-(1,5-dihydroxy-4-pyridon-2-carboxamido)acetamido]-3-(1-methyltetrazol-5-yl)thiomethyl-ceph-3-em-4-carboxylat.

**14.** (6R,7R)7-[(RS)2-(2-Aminothiazol-4-yl)-2-(1,5-dihydroxy-4-pyridon-2-carboxamido)acetamido]-3-(benzothiazol-2-yl)thiomethyl-ceph-3-em-4-carboxylat.

**15.** (6R,7R)7-[(RS)2-(2-Aminothiazol-4-yl)-2-(1,5-dihydroxy-4-pyridon-2-carboxamido)acetamido]-3-[1-(2-fluorethyl)pyridinium-4-yl]thiomethyl-ceph-3-em-4-carboxylat.

**16.** (6R,7R)7-[(RS)2-(2-Aminothiazol-4-yl)-2-(1,5-dihydroxy-4-pyridon-2-carboxamido)acetamido]-3-(pyridin-4-yl)thiomethyl-ceph-3-em-4-carboxylat.

**17.** Antibakterielles, pharmazeutisches Mittel, enthaltend eine wirksame Menge einer Verbindung nach Anspruch 1 und einen pharmazeutisch verträglichen Träger.

**Revendications**

1. Nouveaux composés de céphalosporines représentés par la formule (I) suivante, et leurs sels pharmaceutiquement acceptables,

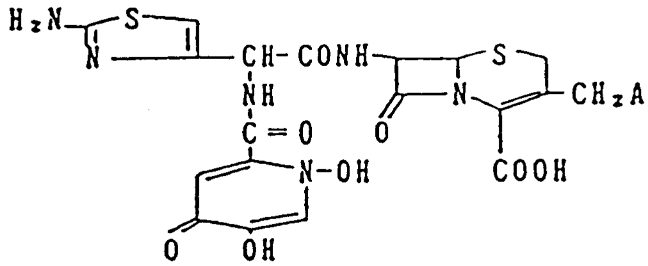

(I)

où A représente un groupe alcanoyloxy comportant 2-5 atomes de carbone, un groupe carbamoyloxy, un groupe azido ou un groupe pyridylthio non substitué ou substitué répondant à la formule (I-1) :

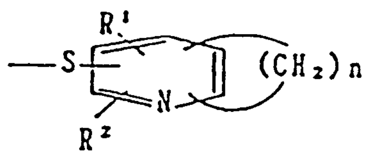

(I-1)

(où n vaut 0 ou représente un nombre entier valant de 3 à 5, $R^1$ et $R^2$ peuvent être identiques ou différents et représentent chacun un atome d'hydrogène, un atome d'halogène, un groupe carboxyle ou un groupe alcoyle inférieur éventuellement substitué par un halogène, comportant 1-5 atomes de carbone), ou un groupe pyridiniumthio non substitué ou substitué répondant à la formule (I-2) :

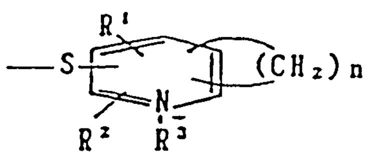

(I-2)

[où n, $R^1$ et $R^2$ ont chacun la même signification que cidessus, $R^3$ représente un groupe alcoyle linéaire ou ramifié comportant 1-5 atomes de carbone, un groupe alcoyle substitué par un halogène, un groupe cyclopropyle, un groupe cyclopropylméthyle, un groupe alcényle, un atome d'oxygène ou un groupe $-(CH_2)_m-B$ (m est un nombre entier valant de 0 à 3, et B représente un groupe hydroxyle, un groupe alcoxy, un groupe amino, un groupe amino substitué par un groupe alcoyle, un groupe carboxyle, un groupe carbamoyle, un groupe acide sulfonique, un groupe amide d'acide sulfonique, un groupe acide hydroxamique, un groupe cyano, un groupe thiol, un groupe alcoylthio, un groupe méthanesulfonylaminocarbonyle ou un groupe acétamidosulfonyle)], ou un groupe pyridinium non substitué ou substitué répondant à la formule (I-3) :

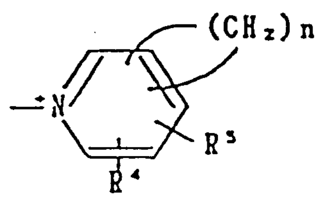

(I-3)

(où n a la même signification que ci-dessus, $R^4$ et $R^5$ peuvent être identiques ou différents et représentent chacun un atome d'hydrogène, un groupe alcoyle linéaire ou ramifié comportant 1-5 atomes de carbone, un groupe carboxyle, un groupe carbamoyle, un groupe acide sulfonique, un groupe amide d'acide sulfonique, un groupe alcoylthio linéaire ou ramifié comportant 1 à 5 atomes de carbone, un groupe alcoylthio substitué par un halogène, un groupe cycloalcanothio, un groupe

cycloalcanométhylthio, un groupe carboxyalcoylthio, un groupe carbamoylalcoylthio, un groupe alcoxyalcoylthio ou un groupe aminoalcoylthio substitué par un groupe alcoyle), ou un groupe thio hétérocyclique ou thio bicyclohétérocyclique à 5 ou 6 chaînons, répondant à la formule (I-4) :

-S-Het (I-4)

(où Het représente un groupe thiazole éventuellement substitué, isothiazole, 1,2,3-thiadiazole, 1,3,4-thiadiazole, 1,3,4-triazole, 1,2,3,4-tétrazole, pyrimidine, 1,2,4-triazine, benzothiazole, benzimidazole, benzoxazole, 1,3,4-triazaindolidine ou 2,3-dihydro-1H-indolidinium).

**2.** (6R,7R)7-[(RS)2-(2-aminothiazol-4-yl)-2-(1,5-dihydroxy-4-pyridone-2-carboxamido)acétamido]-3-[1-(2-hydro xy)éthylpyridinium-4-yl]thiométhyl-céph-3-ème-4-carboxylate.

**3.** (6R,7R)7-[(RS)2-(2-aminothiazol-4-yl)-2-(1,5-dihydroxy-4-pyridone-2-carboxamido)acétamido]-3-(1-cyclopropyl-pyridinium-4-yl)thiométhyl-céph-3-ème-4-carboxylate.

**4.** (6R,7R)7-[(RS)2-(2-aminothiazol-4-yl)-2-(1,5-dihydroxy-4-pyridone-2-carboxamido)acétamido]-3-(1-méthylpyridinium-4-yl)thiométhyl-céph-3-ème-4-carboxylate.

**5.** (6R,7R)7-[(RS)2-(2-aminothiazol-4-yl)-2-(1,5-dihydroxy-4-pyridone-2-carboxamido)acétamido]-3-(1-carboxyméthyl-pyridinium-4-yl)thiométhyl-céph-3-ème-4-carboxylate.

**6.** (6R,7R)7-[(RS)2-(2-aminothiazol-4-yl)-2-(1,5-dihydroxy-4-pyridone-2-carboxamido)acétamido]-3-(1-carbamoylméthyl-pyridinium-4-yl)thiométhyl-céph-3-ème-4-carboxylate.

**7.** (6R,7R)7-[(RS)2-(2-aminothiazol-4-yl)-2-(1,5-dihydroxy-4-pyridone-2-carboxamido)acétamido]-3-(2,3-cyclopenténo-1-carboxyméthyl-pyridinium-4-yl)thiométhyl-céph-3-ème-4-carboxylate.

**8.** (6R,7R)7-[(RS)2-(2-aminothiazol-4-yl)-2-(1,5-dihydroxy-4-pyridone-2-carboxamido)acétamido]-3-(1-méthylpyridinium-3-yl)thiométhyl-céph-3-ème-4-carboxylate.

**9.** (6R,7R)7-[(RS)2-(2-aminothiazol-4-yl)-2-(1,5-dihydroxy-4-pyridone-2-carboxamido)acétamido]-3-[1-(2-sulfamoyl)éthylpyridinium-4-yl]thiométhyl-céph-3-ème-4-carboxylate.

**10.** (6R,7R)7-[(RS)2-(2-aminothiazol-4-yl)-2-(1,5-dihydroxy-4-pyridone-2-carboxamido)acétamido]-3-(1,2,3-thiadiazol-5-yl)thiométhyl-céph-3-ème-4-carboxylate.

**11.** (6R,7R)7-[(RS)2-(2-aminothiazol-4-yl)-2-(1,5-dihydroxy-4-pyridone-2-carboxamido)acétamido]-3-(2,3-cyclopenténo-pyridinium-4-yl)méthyl-céph-3-ème-4-carboxylate.

**12.** (6R,7R)7-[(RS)2-(2-aminothiazol-4-yl)-2-(1,5-dihydroxy-4-pyridone-2-carboxamido)acétamido]-3-(4-méthylthiopyridinium-4-yl)méthyl-céph-3-ème-4-carboxylate.

**13.** (6R,7R)7-[(RS)2-(2-aminothiazol-4-yl)-2-(1,5-dihydroxy-4-pyridone-2-carboxamido)acétamido]-3-(1-méthyltétrazol-5-yl)thiométhyl-céph-3-ème-4-carboxylate.

**14.** (6R,7R)7-[(RS)2-(2-aminothiazol-4-yl)-2-(1,5-dihydroxy-4-pyridone-2-carboxamido)acétamido]-3-(benzothiazol-2-yl)thiométhyl-céph-3-ème-4-carboxylate.

**15.** (6R,7R)7-[(RS)2-(2-aminothiazol-4-yl)-2-(1,5-dihydroxy-4-pyridone-2-carboxamido)acétamido]-3-[1-(2-fluoroéthyl)pyridinium-4-yl]thiométhyl-céph-3-ème-4-carboxylate.

**16.** (6R,7R)7-[(RS)2-(2-aminothiazol-4-yl)-2-(1,5-dihydroxy-4-pyridone-2-carboxamido)acétamido]-3-(pyridine-4-yl)thiométhyl-céph-3-ème-4-carboxylate.

**17.** Composition pharmaceutique antibactérienne contenant une quantité efficace d'un composé selon la revendication 1 et un excipient pharmaceutiquement acceptable.